# EUROPEAN PATENT SPECIFICATION

(11) **EP 2 993 474 B1**
(45) Date of publication and mention of the grant of the patent: **12.06.2019**
(21) Application number: 15173589.1
(22) Date of filing: 23.06.2006
(51) Int. Cl.: G01N 33/574, G01N 33/68, A61K 39/00

(54) **BIOMARKERS FOR OVARIAN CANCER: BETA-2 MICROGLOBULIN**
BIOMARKER FÜR OVARIALKARZINOM: BETA-2-MIKROGLOBULIN
BIOMARQUEURS POUR LE CANCER DE L'OVAIRE: LA BETA-2-MICROGLOBULINE

(30) Priority: 24.06.2005 US 693679 P
(43) Date of publication of application: 09.03.2016
(62) Divisional of application: 06785526.2
(73) Proprietor: Vermillion, Inc., Austin, TX 78731 (US)
(72) Inventor: FUNG, Eric Thomas, Los Altos, CA 94022 (US); DALMASSO, Enrique, Fremont, CA 94536 (US); PODUST, Vladimir, Fremont, CA 94538 (US); WANG, Zheng, Fremont, CA 94538 (US)
(74) Representative: Hiebl, Inge Elisabeth

(56) References cited:
- US-A1- 2005 059 013
- TROPE C G ET AL: "[beta]2-Microglobulin: A tumor marker of gynecologic cancer", AMERICAN JOURNAL OF OBSTETRICS AND GYNECOLOGY 1980 UNITED STATES, vol. 137, no. 6, 1980, pages 743-744, XP009081535,
- HAMPEL D J ET AL: "TOWARD PROTEOMICS IN UROSCOPY: URINARY PROTEIN PROFILES AFTER RADIOCONTRAST MEDIUM ADMINISTRATION", JOURNAL OF THE AMERICAN SOCIETY OF NEPHROLOGY, WILLIAMS AND WILKINS, BALTIMORE, MD, US, vol. 12, no. 5, May 2001 (2001-05), pages 1026-1035, XP001068679, ISSN: 1046-6673
- Alex J Rai ET AL: "518 Arch Pathol Lab Med-Vol 126, December 2002 Biomarkers for Ovarian Cancer-Rai et al Proteomic Approaches to Tumor Marker Discovery Identification of Biomarkers for Ovarian Cancer", Arch Pathol Lab Med, 1 January 2002 (2002-01-01), pages 1518-1526, XP055237228, Retrieved from the Internet: URL:http://www.archivesofpathology.org/doi /pdf/10.1043/0003-9985(2002)126<1518:PATTM D>2.0.CO;2 [retrieved on 2015-12-17]

## Description

### RELATED APPLICATIONS

### FIELD OF THE INVENTION

The invention provides for biomarkers important in the detection of ovarian cancer. The markers were identified by distinguishing the serum protein profile in ovarian cancer patients from healthy individuals using SELDI analysis. The present invention relates the biomarkers to a system and method in which the biomarkers are used for the qualification of ovarian cancer status. The present invention also identifies some of the biomarkers as known proteins.

### BACKGROUND OF THE INVENTION

Ovarian cancer is among the most lethal gynecologic malignancies in developed countries. Annually in the United States alone, approximately 23,000 women are diagnosed with the disease and almost 14,000 women die from it. (Jamal, A., et al., CA Cancer J. Clin, 2002; 52:23-47). Despite progress in cancer therapy, ovarian cancer mortality has remained virtually unchanged over the past two decades. (Id.) Given the steep survival gradient relative to the stage at which the disease is diagnosed, early detection remains the most important factor in improving long-term survival of ovarian cancer patients.

The poor prognosis of ovarian cancer diagnosed at late stages, the cost and risk associated with confirmatory diagnostic procedures, and its relatively low prevalence in the general population together pose extremely stringent requirements on the sensitivity and specificity of a test for it to be used for screening for ovarian cancer in the general population.

The identification of tumor markers suitable for the early detection and diagnosis of cancer holds great promise to improve the clinical outcome of patients. It is especially important for patients presenting with vague or no symptoms or with tumors that are relatively inaccessible to physical examination. Despite considerable effort directed at early detection, no cost effective screening tests have been developed (Paley PJ., Curr Opin Oncol, 2001;13(5):399-402) and women generally present with disseminated disease at diagnosis. (Ozols RF, et al., Epithelial ovarian cancer. In: Hoskins WJ, Perez CA, Young RC, editors. Principles and Practice of Gynecologic Oncology. 3rd ed. Philadelphia: Lippincott, Williams and Wilkins; 2000. p. 981-1057).

The best-characterized tumor marker, CA125, is negative in approximately 30-40% of stage I ovarian carcinomas and its levels are elevated in a variety of benign diseases. (Meyer T, et al., Br J Cancer, 2000;82(9):1535-8; Buamah P., J Surg Oncol, 2000;75(4):264-5; Tuxen MK, et al., Cancer Treat Rev, 1995;21(3):215-45). Its use as a population-based screening tool for early detection and diagnosis of ovarian cancer is hindered by its low sensitivity and specificity. (MacDonald ND, et al., Eur J Obstet Gynecol ReprodBiol, 1999;82(2):155-7; Jacobs I, et al., Hum Reprod, 1989;4(1):1-12; Shih I-M, et al., Tumor markers in ovarian cancer. In: Diamandis EP, Fritsche, H., Lilja, H., Chan, D.W., and Schwartz, M., editor. Tumor markers physiology, pathobiology, technology and clinical applications. Philadelphia: AACC Press; in press). Although pelvic and more recently vaginal sonography has been used to screen high-risk patients, neither technique has the sufficient sensitivity and specificity to be applied to the general population. (MacDonald ND, et al., supra). Recent efforts in using CA125 in combination with additional tumor markers (Woolas RP XF, et al., J Natl Cancer Inst, 1993;85(21):1748-51; Woolas RP, et al., Gynecol Oncol, 1995;59(1):111-6; Zhang Z, et al., Gynecol Oncol, 1999;73(1):56-61; Zhang Z, et al., Use of Multiple Markers to Detect Stage I Epithelial Ovarian Cancers: Neural Network Analysis Improves Performance. American Society of Clinical Oncology 2001; Annual Meeting, Abstract) in a longitudinal risk of cancer model (Skates SJ, et al., Cancer, 1995;76(10 Suppl):2004-10), and in tandem with ultrasound as a second line test (Jacobs I DA, et al., Br Med J, 1993;306(6884):1030-34; Menon U TA, et al., British Journal of Obstetrics and Gynecology, 2000;107(2):165-69) have shown promising results in improving overall test specificity, which is critical for a disease such as ovarian cancer that has a relatively low prevalence.

Due to the dismal prognosis of late stage ovarian cancer, it is the general consensus that a physician will accept a test with a minimal positive predictive value of 10%. (Bast, R.C., et al., Cancer Treatment and Research, 2002; 107:61-97). Extending this to the general population, a general screening test would require a sensitivity greater than 70% and a specificity of 99.6%. Currently, none of the existing serologic markers, such as CA125, CA72-4, or M-CSF, individually delivers such a performance. (Bast, R.C., et al., Int J Biol Markers, 1998; 13:179-87).

Thus, there is a critical need for new serological markers that individually or in combination with other markers or diagnostic modalities deliver the required sensitivity and specificity for early detection of ovarian cancer. (Bast RC, et al., Early detection of ovarian cancer: promise and reality. Ovarian Cancer: ISIS Medical Media Ltd., Oxford, UK; 2001). Without an acceptable screening test, early detection remains the most critical factor in improving long-term survival of patients with ovarian cancer.

Thus, it is desirable to have a reliable and accurate method of determining the ovarian cancer status in patients, the results of which can then be used to manage subject treatment.
TROPE C G ET AL, "[beta]2-Microglobulin: A tumor marker of gynecologic cancer", AMERICAN JOURNAL OF OBSTETRICS AND GYNECOLOGY 1980 UNITED STATES, (1980), vol. 137, no. 6, pages 743 - 744, XP009081535, discloses that β₂-microglobulin is a marker for ovarian cancer.
US 2005/0059013 discloses a method of qualifying ovarian cancer status in a subject comprising: (a) measuring at least one biomarker in a sample from the subject and (b) correlating the measurement with ovarian cancer status. The invention further relates to kits for qualifying ovarian cancer status in a subject.Alex J Rai ET AL, "518 Arch Pathol Lab Med-Vol 126, December 2002 Biomarkers for Ovarian Cancer-Rai et al Proteomic Approaches to Tumor Marker Discovery Identification of Biomarkers for Ovarian Cancer", Arch Pathol Lab Med, (2002-01-01), pages 1518 - 1526, URL: http://www.archivesofpathology.org/doi/pdf710.1043/0003-9985(2002)126 1518:PATTMD2.0.CO;2, XP055237228, discloses that the combined use of bioinformatics tools and proteomic profiling provides an effective approach to screen for potential tumor markers. Comparison of plasma profiles from patients with and without known ovarian cancer uncovered a panel of potential biomarkers for detection of ovarian cancer with discriminatory power complementary to that of CA125.

### BRIEF SUMMARY OF THE INVENTION

The present invention provides sensitive and quick methods that are useful for determining the ovarian cancer status by measuring these markers. The measurement of these markers in patient samples provides information that diagnosticians can correlate with a probable diagnosis of human cancer or a negative diagnosis (e.g., normal or disease-free). The markers are characterized by molecular weight and/or by their known protein identities. The markers can be resolved from other proteins in a sample by using a variety of fractionation techniques, e.g., chromatographic separation coupled with mass spectrometry, protein capture using immobilized antibodies or by traditional immunoassays. In preferred embodiments, the method of resolution involves Surface-Enhanced Laser Desorption/Ionization ("SELDI") mass spectrometry, in which the surface of the mass spectrometry probe comprises adsorbents that bind the markers.

More specifically, the biomarkers identified in Table 1 were discovered, some of which were identified, in accordance with the methods described herein. Those biomarkers that were identified include platelet factor 4 (PF4), β-2-microglobin, albumin, Vitamin D binding protein, transthyretin/albumin complex and transferrin/ApoA1 complex.

The present invention provides an in vitro method for diagnosing ovarian cancer in a subject comprising: (a) measuring β-2 microglobulin, CA125, ApoAl, transthyretin and transferrin in a biological sample from the subject; and (b) correlating the measurement with ovarian cancer, wherein an increase in β-2 microglobulin and CA125 and a decrease in ApoAl, transthyretin and transferrin relative to a respective reference measurement for each of said biomarkers is indicative of ovarian cancer. In certain methods, the measuring step comprises detecting the presence or absence of markers in the sample. In other methods, the measuring step comprises quantifying the amount of marker(s) in the sample. In other methods, the measuring step comprises qualifying the type of biomarker in the sample.

The invention also relates to methods wherein the measuring step comprises: providing a subject sample of blood or a blood derivative; fractionating proteins in the sample on an anion exchange resin and collecting fractions that contain the biomarkers as defined above from the fractions on a surface of a substrate comprising capture reagents that bind the protein biomarkers. The blood derivative is, e.g., serum or plasma. In preferred embodiments, the substrate is a SELDI probe comprising an IMAC copper surface and wherein the protein biomarkers are detected by SELDI. In other embodiments, the substrate is a SELDI probe comprising biospecific affinity reagents that bind the biomarkers and wherein the protein biomarkers are detected by SELDI. In other embodiments, the substrate is a microtiter plate comprising biospecific affinity reagents that bind biomarkers and the protein biomarkers are detected by immunoassay.

Further described are methods which further comprise managing subject treatment based on the status determined by the method. For example, if the result of the methods of the present invention is inconclusive or there is reason that confirmation of status is necessary, the physician may order more tests. Alternatively, if the status indicates that surgery is appropriate, the physician may schedule the patient for surgery. Likewise, if the result of the test is positive, e.g., the status is late stage ovarian cancer or if the status is otherwise acute, no further action may be warranted. Furthermore, if the results show that treatment has been successful, no further management may be necessary.

Described are such methods where the at least one biomarker is measured again after subject management. In these instances, the step of managing subject treatment is then repeated and/or altered depending on the result obtained.

The term "ovarian cancer status" refers to the status of the disease in the patient. Examples of types of ovarian cancer statuses include, but are not limited to, the subject's risk of cancer, the presence or absence of disease, the stage of disease in a patient, and the effectiveness of treatment of disease. Other statuses and degrees of each status are known in the art.

Further described are methods further comprising measuring at least one previously known ovarian cancer biomarker in a sample from the subject and correlating measurement of the previously known ovarian cancer biomarker and the measurement of one or more of the twenty-seven biomarkers of Table 1, e.g., β-2 microglobulin, with ovarian cancer status. In certain variants only one additional biomarker is measured, in addition to one or more markers selected from Table 1 above, while in other embodiments more than one previously known ovarian cancer biomarker is measured.

Examples of previously known ovarian cancer biomarkers, e.g., but are not limited to, CA125, CA125 II, CA15-3, CA19-9, CA72-4, CA 195, tumor associated trypsin inhibitor (TATI), CEA, placental alkaline phosphatase (PLAP), Sialyl TN, galactosyltransferase, macrophage colony stimulating factor (M-CSF, CSF-1), lysophosphatidic acid (LPA), 110 kD component of the extracellular domain of the epidermal growth factor receptor (p110EGFR), tissue kallikreins, e.g., kallikrein 6 and kallikrein 10 (NES-1), prostasin, HE4, creatine kinase B (CKB), LASA, HER-2/neu, urinary gonadotropin peptide, Dianon NB 70/K, Tissue peptide antigen (TPA), osteopontin and haptoglobin, bikunin, MUC1, and protein variants (e.g., cleavage forms, isoforms) of the markers. Additionally, those biomarkers identified in Table 3 are useful ovarian cancer biomarkers.

In certain variants, the method provides for the measurement of a subset of the twenty-seven biomarkers of Table 1 above. In another variant, the method provides for the measurement of two biomarkers, albumin and transthyretin (wherein the Apo A1 is selected from unmodified Apo A1 and modified, wherein the thransthyretin is selected from the group consisting of transthyretin ΔN10, native transthyretin, cysteinylated transthyretin, sulfonated transthyretin, CysGly modified transthyretin, and glutathionylated transthyretin). In a preferred variant, the two biomarkers are modified ApoA1 and albumin. In some variants, at least one previously known marker, in a sample from the subject is also measured, and the measurement of the previously known marker and the measurements of a subset of the other twenty-seven biomarkers are correlated with ovarian cancer status.

Further described is a method of qualifying ovarian cancer status in a subject comprising (a) measuring at least one biomarker in a sample from the subject, wherein the biomarker is selected from the group set forth in Table 1 above and combinations thereof, and (b) correlating the measurement with ovarian cancer status. In certain variants, the biomarker is β-2 microglobulin. In certain methods, the measuring step comprises detecting the presence or absence of markers in the sample. In other methods,
the measuring step comprises quantifying the amount of marker(s) in the sample. In other methods, the measuring step comprises qualifying the type of biomarker in the sample.

The accuracy of a diagnostic test is characterized by a Receiver Operating Characteristic curve ("ROC curve"). An ROC is a plot of the true positive rate against the false positive rate for the different possible cutpoints of a diagnostic test. An ROC curve shows the relationship between sensitivity and specificity. That is, an increase in sensitivity will be accompanied by a decrease in specificity. The closer the curve follows the left axis and then the top edge of the ROC space, the more accurate the test. Conversely, the closer the curve comes to the 45-degree diagonal of the ROC graph, the less accurate the test. The area under the ROC is a measure of test accuracy. The accuracy of the test depends on how well the test separates the group being tested into those with and without the disease in question. An area under the curve (referred to as "AUC") of 1 represents a perfect test, while an area of 0.5 represents a less useful test. Thus, preferred biomarkers and diagnostic methods of the present invention have an AUC greater than 0.50, more preferred tests have an AUC greater than 0.60, more preferred tests have an AUC greater than 0.70.

Preferred methods of measuring the biomarkers include use of a biochip array. Biochip arrays useful in the invention include protein and nucleic acid arrays. One or more markers are captured on the biochip array and subjected to laser ionization to detect the molecular weight of the markers. Analysis of the markers is, for example, by molecular weight of the one or more markers against a threshold intensity that is normalized against total ion current. Preferably, logarithmic transformation is used for reducing peak intensity ranges to limit the number of markers detected.

In preferred methods of the present invention, the step of correlating the measurement of the biomarkers with ovarian cancer status is performed by a software classification algorithm. Preferably, data is generated on immobilized subject samples on a biochip array, by subjecting said biochip array to laser ionization and detecting intensity of signal for mass/charge ratio; and, transforming the data into computer readable form; and executing an algorithm that classifies the data according to user input parameters, for detecting signals that represent markers present in ovarian cancer patients and are lacking in non-cancer subject controls.

Preferably the biochip surfaces are, for example, ionic, anionic, comprised of immobilized nickel ions, comprised of a mixture of positive and negative ions, comprised of one or more antibodies, single or double stranded nucleic acids, proteins, peptides or fragments thereof, amino acid probes, or phage display libraries.

In other preferred methods one or more of the markers are measured using laser desorption/ionization mass spectrometry, comprising providing a probe adapted for use with a mass spectrometer comprising an adsorbent attached thereto, and contacting the subject sample with the adsorbent, and; desorbing and ionizing the marker or markers from the probe and detecting the deionized/ionized markers with the mass spectrometer.

Preferably, the laser desorption/ionization mass spectrometry comprises: providing a substrate comprising an adsorbent attached thereto; contacting the subject sample with the adsorbent; placing the substrate on a probe adapted for use with a mass spectrometer comprising an adsorbent attached thereto; and, desorbing and ionizing the marker or markers from the probe and detecting the desorbed/ionized marker or markers with the mass spectrometer.

The adsorbent can for example be hydrophobic, hydrophilic, ionic or metal chelate adsorbent, such as, nickel or an antibody, single- or double stranded oligonucleotide, amino acid, protein, peptide or fragments thereof.

The methods of the present invention can be performed on any type of patient sample that would be amenable to such methods, e.g., blood, serum and plasma.

In certain variants, a plurality of biomarkers in a sample from the subject are measured, wherein the biomarkers are selected from the group set forth in Table 1, e.g., β-2 microglobulin., and at least one known marker. The plurality of biomarkers may consist of albumin/transthyretin complex, and the Apo A1/albumin complex. The measurement of the plurality of biomarkers can also include measuring at least one previously known ovarian cancer biomarker. Preferably, the protein biomarkers are measured by SELDI or immunoassay.

Further described is a method comprising measuring at least one biomarker in a sample from the subject, wherein the biomarker is selected from the group set forth in Table 1 above and combinations thereof. The method may further comprise measuring albumin/Apo A1 complex and/or at least one known ovarian cancer marker, i.e., Marker 4, e.g., CA125, CA125 II, CA15-3, CA19-9, CA72-4, CA 195, TATI, CEA, PLAP, Sialyl TN, galactosyltransferase, M-CSF, CSF-1, LPA, p110EGFR, tissue kallikreins, prostasin, HE4, CKB, LASA, HER-2/neu, urinary gonadotropin peptide, Dianon NB 70/K, TPA, osteopontin and haptoglobin, bikunin, MUC1, and protein variants (e.g., cleavage forms, isoforms) of the markers.

Further described are kits comprising (a) a capture reagent that binds a biomarker selected from Table 1, and combinations thereof; and (b) a container comprising at least one of the biomarkers. In variants, the capture reagent binds a plurality of the biomarkers. In one variant, the plurality comprises albumin/Apo A1 complex and transthyretin/albumin complex. While the capture reagent can be any type of reagent, preferably the reagent is a SELDI probe. The capture reagent may also bind other known biomarkers, e.g., one or more of the biomarkers identified in Table 3. In certain variants, the kit of further comprises a second capture reagent that binds one of the biomarkers that the first capture reagent does not bind.

Further kits as disclosed comprise (a) a first capture reagent that binds at least one biomarker selected from Table 1, and (b) a second capture reagent that binds at least one of the biomarkers that is not bound by the first capture reagent. Preferably, at least one the capture reagent is an antibody. Certain kits further comprise an MS probe to which at least one capture reagent is attached or is attachable.

In certain kits, the capture reagent comprises an immobilized metal chelate ("IMAC").

Certain kits further comprise a wash solution that selectively allows retention of the bound biomarker to the capture reagent as compared with other biomarkers after washing.

Further described are kits comprising (a) a first capture reagent that binds at least one biomarker selected from Table 1, and (b) instructions for using the capture reagent to measure the biomarker. In certain of these kits, the capture reagent comprises an antibody. Furthermore, some kits further comprise an MS probe to which the capture reagent is attached or is attachable. In some kits, the capture reagent comprises an IMAC. The kits may also contain a wash solution that selectively allows retention of the bound biomarker to the capture reagent as compared with other biomarkers after washing. Preferably, the kit comprises written instructions for use of the kit for determining ovarian cancer status and the instructions provide for contacting a test sample with the capture reagent and measuring one or more biomarkers retained by the capture reagent.

The kit also provides for a capture reagent, which is an antibody, single or double stranded oligonucleotide, amino acid, protein, peptide or fragments thereof.

Measurement of one or more protein biomarkers using the kit is suitably by mass spectrometry or immunoassays such as an ELISA.

Purified proteins for detection of ovarian cancer and/or generation of antibodies for further diagnostic assays are also described. Purified proteins include a purified peptide of any of the markers set forth in Table 1 above. Also described is this purified peptide further comprising a detectable label.

Further described is an article manufacture comprising at least one capture reagent bound to at least two biomarkers selected from Table 1. Other variants of the article of manufacture further comprise a capture reagent that binds other known ovarian cancer markers, e.g., but not limited to, CTAP3, CA125, CA125 II, CA15-3, CA19-9, CA72-4, CA 195, TATI, CEA, PLAP, Sialyl TN, galactosyltransferase, M-CSF, CSF-1, LPA, p110EGFR, tissue kallikreins, prostasin, HE4, CKB, LASA, HER-2/neu, urinary gonadotropin peptide, Dianon NB 70/K, TPA, osteopontin and haptoglobin, bikunin, MUC1 and protein variants (e.g., cleavage forms, isoforms) of the markers.

Further described is a system comprising a plurality of capture reagents each of which has bound to it a different biomarker selected from a markers of Table 1 and at least one previously known biomarker.

In another variant, non-invasive medical imaging techniques such as transvaginal ultrasound, positron emisson tomography (PET) or single photon emission computerized tomography (SPECT) imaging are particularly useful for the detection of cancer, coronary artery disease and brain disease. Ultrasound with Doppler flow, PET, and SPECT imaging show the chemical functioning of organs and tissues, while other imaging techniques-such as X-ray, CT and MRI-primarily show structure. The use of ultrasound with flow, PET and SPECT imaging has become increasingly useful for qualifying and monitoring the development of diseases such as ovarian cancer.

The peptide biomarkers disclosed herein, or fragments thereof, can be used in the context of PET and SPECT imaging applications. After modification with appropriate tracer residues for PET or SPECT applications, peptide biomarkers that interact with tumor proteins can be used to image the deposition of biomarkers in ovarian cancer patients.

Other aspects of the invention are described *infra*.
The invention further relates to the following embodiments:
**1.** A method for qualifying ovarian cancer status in a subject comprising:
   (a) measuring at least one biomarker in a biological sample from the subject, wherein the at least one biomarker is selected from the group consisting of the biomarkers of Table 1; and
   (b) correlating the measurement with ovarian cancer status.
**2.** The method of item 1, wherein the at least one biomarker of Table 1 is β-2 microglobulin.
**3.** The method of item **2,** wherein the at least one biomarker is measured by capturing the biomarker on an adsorbent surface of a SELDI probe and detecting the captured biomarkers by laser desorption-ionization mass spectrometry.
**4.** The method of item **2,** wherein the at least one biomarker is measured by immunoassay.
**5.** The method of item **2,** wherein the sample is serum.
**6.** The method of item **2,** wherein the correlating is performed by a software classification algorithm.
**7.** The method of item **2,** wherein ovarian cancer status is selected from ovarian cancer and non-ovarian cancer.
**8.** The method of item **2,** wherein ovarian cancer status is selected from early stage and late stage.
**9.** The method of item **2,** further comprising: (c) managing subject treatment based on the status.
**10.** The method of item **3,** wherein the adsorbent is a IMAC copper adsorbent.
**11.** The method of item **3,** wherein the adsorbent is a biospecific adsorbent.
**12.** The method of item **7,** wherein, if the measurement correlates with ovarian cancer, then managing subject treatment comprises administering an inhibitor of any of the up-regulated biomarkers of Table 1 to the subject.
**13.** The method of item **7,** further comprising: (d) measuring the at least one biomarker after subject management and correlating the measurement with disease progression.
**14.** A method for determining the course of ovarian cancer comprising:
   (a) measuring, at a first time, at least one biomarker in a biological sample from the subject, wherein the at least one biomarker is selected from the group consisting of the biomarkers of Table 1;
   (b) measuring, at a second time, the at least one biomarker in a biological sample from the subject; and
   (c) comparing the first measurement and the second measurement; wherein the comparative measurements determine the course of the ovarian cancer.
**15.** The method of item **14,** wherein the biomarker is β-2 microglobulin.
**16.** A method comprising measuring at least one biomarker in a sample from a subject, wherein the at least one biomarker is selected from the group consisting of biomarkers of Table 1.
**17.** The method of item **16,** wherein the biomarker is β-2 microglobulin.
**18.** A composition comprising a purified biomolecule selected from the biomarkers of Table 1.
**19.** A composition comprising a biospecific capture reagent that specifically binds a biomolecule selected from the biomarkers of Table 1.
**20.** The method of item **19,** wherein the biomarker is β-2 microglobulin.
**21.** The composition of item **20** wherein the biospecific capture reagent is an antibody.
**22.** The composition of item **20** wherein the biospecific capture reagent is bound to a solid support.
**23.** A composition comprising a biospecific capture reagent bound to a biomarker of Table 1.
**24.** A kit comprising:
   (a) a solid support comprising at least one capture reagent attached thereto, wherein the capture reagent binds at least one biomarker from a first group consisting of the Biomarkers of Table 1; and
   (b) instructions for using the solid support to detect a biomarker of Table 1.
**25.** The kit of item **24,** wherein the biomarker is β-2 microglobulin.
**26.** The kit of item **25,** wherein the solid support comprising a capture reagent is a SELDI probe.
**27.** The kit of item **25,** wherein the capture reagent is a IMAC copper adsorbent.
**28.** The kit of item **25** additionally comprising: (c) a container containing at least one of the biomarkers of Table **1.**
**29.** The kit of item **25** additionally comprising: (c) a biospecific chromatography sorbent.
**30.** A kit comprising:
   (a) a solid support comprising at least one capture reagent attached thereto, wherein the capture reagents bind at least one biomarker selected from the group consisting of the biomarkers of Table 1; and
   (b) a container containing at least one of the biomarkers.
**31.** The kit of item **24,** wherein the biomarker is β-2 microglobulin.
**32.** The kit of item **31** wherein the solid support comprising a capture reagent is a SELDI probe.
**33.** The kit of items **31** additionally comprising: (c) a biospecific chromatography sorbent.
**34.** The kit of item **31** wherein the capture reagent is a IMAC copper adsorbent.
**35.** A software product comprising:
   a. code that accesses data attributed to a sample, the data comprising measurement of at least one biomarker in the sample, the biomarker selected from the group consisting of the biomarkers of Table 1; and
   b. code that executes a classification algorithm that classifies the ovarian cancer status of the sample as a function of the measurement.
**36.** A method comprising detecting a biomarker of Table 1 by mass spectrometry or immunoassay.
**37.** A method comprising communicating to a subject a diagnosis relating to ovarian cancer status determined from the correlation of biomarkers in a sample from the subject, wherein said biomarkers are selected from Table 1.
**38.** The method of item **37** wherein the diagnosis is communicated to the subject via a computer-generated medium.
**39.** A method for identifying a compound that interacts with biomarker of table 1, wherein said method comprises:
   a) contacting a biomarker of Table 1 with a test compound; and
   b) determining whether the test compound interacts with the biomarker of Table 1.
**40.** A method for modulating the concentration of a biomarker of Table 1 in a cell, wherein said method comprises:
   a) contacting said cell with a small molecule, wherein said small molecule inhibits expression of a biomarker of Table 1.
**41.** A method of treating a condition in a subject, wherein said method comprises:
   administering to a subject a therapeutically effective amount of a small molecule, wherein said small molecule inhibits expression of a biomarker of Table 1.
**42.** The method of item **41** wherein said condition is ovarian cancer.

### BRIEF DESCRIPTION OF THE DRAWINGS

FIG. 1 (includes FIG. 1A through IE) shows mass spectra of the specified markers. The mass spectral peak of the marker is designated within the depicted spectra with a vertical line.
FIG. 2 sets for the amino acid sequence of β-2-microglobin (SwissProt Accession Number P61769) (SEQ ID NO:5).

### DETAILED DESCRIPTION OF THE INVENTION

### 1. INTRODUCTION

A biomarker is an organic biomolecule which is differentially present in a sample taken from a subject of one phenotypic status (e.g., having a disease) as compared with another phenotypic status (e.g., not having the disease). A biomarker is differentially present between different phenotypic statuses if the mean or median expression level of the biomarker in the different groups is calculated to be statistically significant. Common tests for statistical significance include, among others, t-test, ANOVA, Kruskal-Wallis, Wilcoxon, Mann-Whitney and odds ratio. Biomarkers, alone or in combination, provide measures of relative risk that a subject belongs to one phenotypic status or another. Therefore, they are useful as markers for disease (diagnostics), therapeutic effectiveness of a drug (theranostics) and drug toxicity.

### 2. BIOMARKERS FOR OVARIAN CANCER

### 2.1. Biomarkers

This invention provides polypeptide-based biomarkers that are differentially present in subjects having ovarian cancer, in particular, ovarian cancer versus normal (non-ovarian cancer). The biomarkers of this invention are differentially present depending on ovarian cancer status, including, early stage ovarian cancer versus healthy controls, early stage ovarian cancer versus post-operative cancer free (serial samples from patients before and after treatment), and early stage ovarian cancer versus benign disease, either ovarian or non-ovarian disease. The biomarkers are characterized by mass-to-charge ratio as determined by mass spectrometry, by the shape of their spectral peak in time-of-flight mass spectrometry and by their binding characteristics to adsorbent surfaces. These characteristics provide one method to determine whether a particular detected biomolecule is a biomarker of this invention as defined above. These characteristics represent inherent characteristics of the biomolecules and not process limitations in the manner in which the biomolecules are discriminated. In one aspect, this invention provides these biomarkers as defined above in isolated form.

The biomarkers were discovered using SELDI technology employing ProteinChip arrays from Ciphergen Biosystems, Inc. (Fremont, CA) ("Ciphergen"). Serum samples were collected from subjects diagnosed with ovarian cancer and subjects diagnosed as normal. The samples were fractionated by anion exchange chromatography. Fractionated samples were applied to SELDI biochips and spectra of polypeptides in the samples were generated by time-of-flight mass spectrometry on a Ciphergen PBSII mass spectrometer. The spectra thus obtained were analyzed by Ciphergen Express^{tm} Data Manager Software with Biomarker Wizard and Biomarker Pattern Software from Ciphergen Biosystems, Inc. The mass spectra for each group were subjected to scatter plot analysis. A Mann-Whitney test analysis was employed to compare ovarian cancer and control groups for each protein cluster in the scatter plot, and proteins were selected that differed significantly (p<0.0001) between the two groups. This method is described in more detail in the Example Section.

The "ProteinChip assay" column in Table 1 refers to chromatographic fraction in which the biomarker is found, the type of biochip to which the biomarker binds and the wash conditions, as per the Examples.

The biomarkers are presented in the following Table 1.

**Table 1**

| **Marker Mass values** | **Identity** | **P-Value** | **Up or down regulated in ovarian cancer** | **ProteinChip® assay** |
|---|---|---|---|---|
| M 3.88 k | | <0.0001 | UP | IMAC-Cu |
| | | | 1vF | |
| M 4.14 k | | <0.0001 | UP | IMAC-Cu |
| | | | 1vF | |
| | | | 1vB | |
| M 4.45 k | | <0.0001 | UP | CM10 (cation) |
| | | | 1vF | |
| | | | 1vB | |
| M 4.48 k | | <0.0001 | UP | IMAC-Cu |
| | | | 1vF | |
| | | | 1vB | |
| M 4.64 k | | <0.0001 | UP | IMAC-Cu |
| | | | 1vF | |
| | | | 1vB | |
| M 4.80 k | | <0.0001 | UP | IMAC-Cu |
| | | | 1vF | |
| | | | 1vB | |
| M 7.70 k | Platelet factor 4 (PF4) | <0.0001 | UP | IMAC-Cu |
| | | | 1vF | |
| | | | 1vB | |
| M 7.90 k | | <0.0001 | UP | IMAC-Cu |
| | | | 1vF | |
| M 9.30 k | | <0.0001 | UP | IMAC-Cu |
| | | | 1vF | |
| | | | 1vB | |
| M 10.50 k | | <0.0001 | DOWN | IMAC-Cu |
| | | | 1vB | |
| M 11.70 k | β-2-microglobin | <0.0001 | UP | IMAC-Cu |
| | | | 1vF | |
| | | | 1vB | |
| M 12.40 k | | <0.0001 | UP | Q10 |
| | | | 1vF | |
| M 22.20 k | albumin | <0.0001 | UP | IMAC-Cu |
| | | | 1vF | |
| | | | 1vB | |
| M 33.30 k | albumin | <0.0001 | UP | Q10 |
| | | | 1vF | |
| M 38.60 k | | <0.0001 | DOWN | MEP CM10 |
| | | | 1vB | |
| M 40.20 k | | <0.0001 | DOWN | Q10 |
| | | | 1vF | |
| | | | 1vB | |
| M 41.60 k | | <0.0001 | DOWN | Q10 |
| | | | 1vF | |
| | | | 1vB | |
| M 44.50 k | | <0.0001 | DOWN | IMAC-Cu |
| | | | 1vF | |
| M 47.30 k | | <0.0001 | DOWN | IMAC-Cu |
| | | | 1vF | |
| | | | 1vB | |
| M 51.10 k | Vitamin D binding protein | <0.0001 | UP | IMAC-Cu |
| | | | 1vF | |
| | | | 1vB | |
| M 66.60 k | albumin | <0.0001 | DOWN | IMAC-Cu |
| | | | 1vF | |
| M 80.00 k | | <0.0001 | DOWN | Q10 |
| | | | 1vF | |
| | | | 1vB | |
| M 94.60 k | | <0.0001 | DOWN | IMAC-Cu |
| | | | 1vF | |
| | | | 1vB | |
| M 100.00 k | albumin | <0.0001 | DOWN | IMAC-Cu |
| | | | 1vF | |
| | | | 1vB | |
| M 107.00 k | transferrin (presumed triple-charged tetramer) | <0.0001 | DOWN | IMAC-Cu |
| | | | 1vF | |
| | | | 1vB | |
| M 132.00 k | | <0.0001 | DOWN | IMAC-Cu |
| | | | 1vF | |
| | | | 1vB | |
| M 146.00 k | | <0.0001 | DOWN | IMAC-Cu |
| | | | 1vF | |
| | | | 1vB | |

| | | | | |
|---|---|---|---|---|
| 1vF = early stage ovarian cancer v. cancer free; 1vB = early stage ovarian v. benign disease (either benign ovarian disease, benign non-ovarian or both). As would be understood, references herein to a biomarker of Table 1 or other similar phrase indicates one or more of the twenty-seven biomarkers as set forth in the above Table 1. As also would be understood, the symbol "k" with respect to the designated mass values is an abbreviation for thousands or kilo-. Specifically identified markers are designated by the peptide(s) listed under the "Identity" column IN Table 1. The theoretical masses of identified markers include 7.92 kD of platelet factor 4, 78.7 kD of transferrin and 28.1 kD of ApoA1. | | | | |

The biomarkers of this invention as defined above are characterized by their mass-to-charge ratio as determined by mass spectrometry. The mass-to-charge ratio of each biomarker is provided in Table 1 after the "M." Thus, for example, the first marker in Table 1 has a measured mass-to-charge ratio of 3886.8. The mass-to-charge ratios were determined from mass spectra generated on a Ciphergen Biosystems, Inc. PBS II mass spectrometer. This instrument has a mass accuracy of about +/- 0.15 percent. Additionally, the instrument has a mass resolution of about 400 to 1000 m/dm, where m is mass and dm is the mass spectral peak width at 0.5 peak height. The mass-to-charge ratio of the biomarkers was determined using Biomarker Wizard^{tm} software (Ciphergen Biosystems, Inc.). Biomarker Wizard assigns a mass-to-charge ratio to a biomarker by clustering the mass-to-charge ratios of the same peaks from all the spectra analyzed, as determined by the PBSII, taking the maximum and minimum mass-to-charge-ratio in the cluster, and dividing by two. Accordingly, the masses provided reflect these specifications. In view of such mass accuracy and resolution variances associated with the mass spectral instrument and operation thereof, the mass of each of markers of Table 1 above should be considered "about" the listed value. It is also intended that such mass accuracy and resolution variances and thus the understood "about" mass values of each of the markers disclosed herein is inclusive of variants of the markers as may exist due to sex, genotype and/or ethnicity of the subject and the particular cancer or origin or stage thereof.

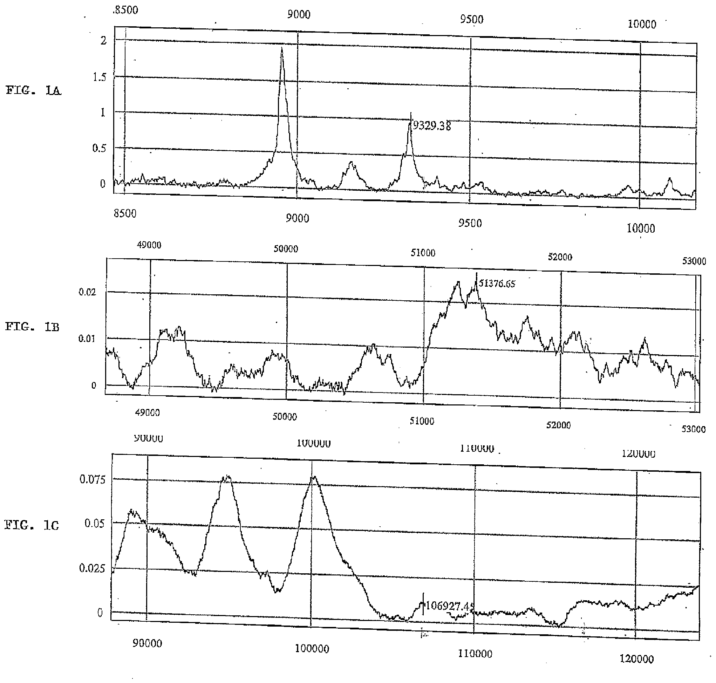

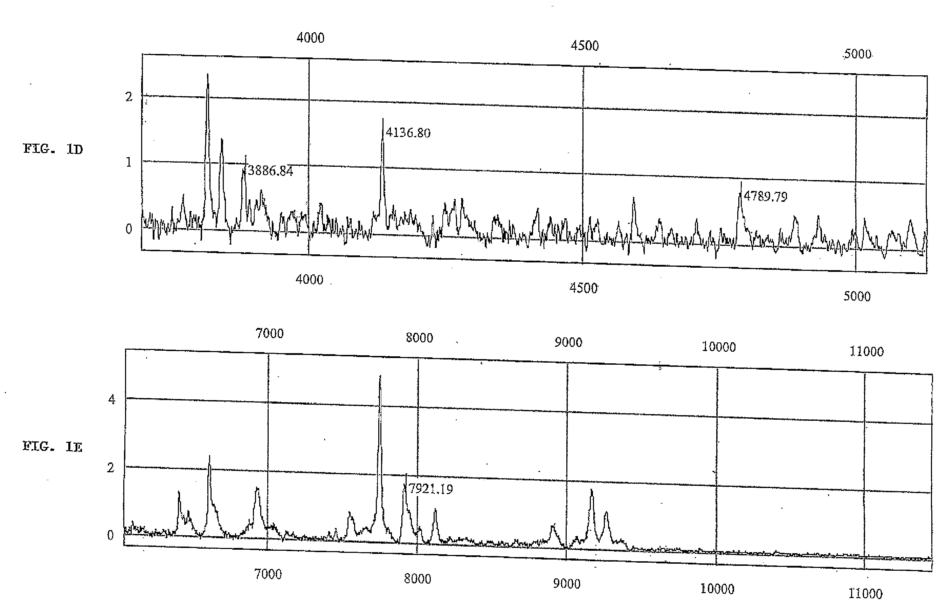

The biomarkers of this invention as defined above are further characterized by the shape of their spectral peak in time-of-flight mass spectrometry. Mass spectra showing peaks representing the biomarkers are presented in FIG. 1. In particular, FIG. 1A shows the peak of M about 9.30 k. FIG. 1B shows the peak of M about 51.10 k, which is Vitamin D binding protein. FIG. 1C shows the peak of M about 107.00 k, which is ApoAl/transferrin complex. FIG. ID shows the peaks of the M about 3.88 k, M about 4.14 k, and M about 4.80 k. FIG. 1E shows the peak of M about 7.90 k.

The biomarkers of this invention as defined above are further characterized by their binding properties on chromatographic surfaces. Most of the biomarkers bind to cation exchange adsorbents (*e*.*g*., the Ciphergen® WCX ProteinChip® array) after washing with 100 mM sodium acetate at pH 4. for IMAC-Cu chips (Ciphergen®), preferred wash includes 100 mM sodium phosphate, pH 7.0.

The specific identity of certain of the biomarkers of this invention as defined above has been determined and is indicated in Table 1. For biomarkers whose identify has been determined, the presence of the biomarker can be determined by other methods known in the art.

In a specific exemplary embodiment of the invention, the biomarker of the is β-2-microglobin (SwissProt Accession Number P61769), as discussed in detail below.

Because the biomarkers of this invention as defined above are characterized by mass-to-charge ratio, binding properties and/or spectral shape, they can be detected by mass spectrometry without knowing their specific identity. However, if desired, biomarkers whose identity is not determined can be identified by, for example, determining the amino acid sequence of the polypeptides. For example, a biomarker can be peptide-mapped with a number of enzymes, such as trypsin or V8 protease, and the molecular weights of the digestion fragments can be used to search databases for sequences that match the molecular weights of the digestion fragments generated by the various enzymes. Alternatively, protein biomarkers can be sequenced using tandem MS technology. In this method, the protein is isolated by, for example, gel electrophoresis. A band containing the biomarker is cut out and the protein is subject to protease digestion. Individual protein fragments are separated by a first mass spectrometer. The fragment is then subjected to collision-induced cooling, which fragments the peptide and produces a polypeptide ladder. A polypeptide ladder is then analyzed by the second mass spectrometer of the tandem MS. The difference in masses of the members of the polypeptide ladder identifies the amino acids in the sequence. An entire protein can be sequenced this way, or a sequence fragment can be subjected to database mining to find identity candidates.

The preferred biological source for detection of the biomarkers is serum. However, in other embodiments, the biomarkers can be detected in serum and urine.

The biomarkers of this invention as defined above are biomolecules. Accordingly, this invention provides these biomolecules in isolated form. The biomarkers can be isolated from biological fluids, such as urine or serum. They can be isolated by any method known in the art, based on both their mass and their binding characteristics. For example, a sample comprising the biomolecules can be subject to chromatographic fractionation, as described herein, and subject to further separation by, e.g., acrylamide gel electrophoresis. Knowledge of the identity of the biomarker also allows their isolation by immunoaffinity chromatography.

### 2.2 β-2 microglobulin

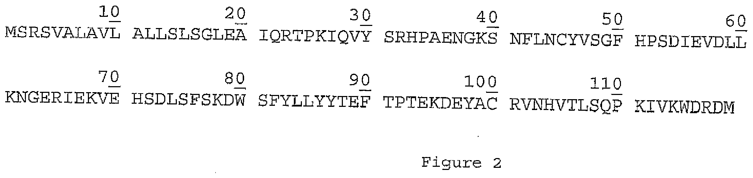

One exemplary biomarker that is used in the methods of the present invention is β2-microglobulin. β2-microglobulin is described as a biomarker for ovarian cancer in US provisional patent publication 60/693,679, filed June 24, 2005 (Fung et al.). The mature for of β2-microglobulin is a 99 amino acid protein derived from an 119 amino acid precursor (GI:179318; SwissProt Accession No. P61769). The amino acid sequence of β-2-microglobin is set forth in Figure 2 (SEQ ID NO:5). The mature form of β2-microglobulin consist of residues 21-119 of SEQ ID NO:5. β2-microglobulin is recognized by antibodies available from, e.g., Abcam (catalog AB759) (www.abcam.com, Cambridge, MA). A specific β2-microglobulin biomarker identified is presented in Table 2.

**TABLE 2**

| **Marker** | **P-Value** | **Up or down regulated in ovarian cancer** | **ProteinChip® assay** |
|---|---|---|---|
| β2-microglobulin (M11.7K) (predicted mass: 11729.17 D) | <0.0001 | Up | IMAC-Cu⁺⁺ |

### 3. BIOMARKERS AND DIFFERENT FORMS OF A PROTEIN

Proteins frequently exist in a sample in a plurality of different forms. These forms can result from either or both of pre- and post-translational modification. Pre-translational modified forms include allelic variants, splice variants and RNA editing forms. Post-translationally modified forms include forms resulting from proteolytic cleavage (e.g., cleavage of a signal sequence or fragments of a parent protein), glycosylation, phosphorylation, lipidation, oxidation, methylation, cysteinylation, sulphonation and acetylation. When detecting or measuring a protein in a sample, the ability to differentiate between different forms of a protein depends upon the nature of the difference and the method used to detect or measure. For example, an immunoassay using a monoclonal antibody will detect all forms of a protein containing the eptiope and will not distinguish between them. However, a sandwich immunoassay that uses two antibodies directed against different epitopes on a protein will detect all forms of the protein that contain both epitopes and will not detect those forms that contain only one of the epitopes. In diagnostic assays, the inability to distinguish different forms of a protein has little impact when the forms detected by the particular method used are equally good biomarkers as any particular form. However, when a particular form (or a subset of particular forms) of a protein is a better biomarker than the collection of different forms detected together by a particular method, the power of the assay may suffer. In this case, it is useful to employ an assay method that distinguishes between forms of a protein and that specifically detects and measures a desired form or forms of the protein. Distinguishing different forms of an analyte or specifically detecting a particular form of an analyte is referred to as "resolving" the analyte.

Mass spectrometry is a particularly powerful methodology to resolve different forms of a protein because the different forms typically have different masses that can be resolved by mass spectrometry. Accordingly, if one form of a protein is a superior biomarker for a disease than another form of the biomarker, mass spectrometry may be able to specifically detect and measure the useful form where traditional immunoassay fails to distinguish the forms and fails to specifically detect to useful biomarker.

One useful methodology combines mass spectrometry with immunoassay. First, a biosepcific capture reagent (e.g., an antibody, aptamer or Affibody that recognizes the biomarker and other forms of it) is used to capture the biomarker of interest. Preferably, the biospecific capture reagent is bound to a solid phase, such as a bead, a plate, a membrane or an array. After unbound materials are washed away, the captured analytes are detected and/or measured by mass spectrometry. (This method also will also result in the capture of protein interactors that are bound to the proteins or that are otherwise recognized by antibodies and that, themselves, can be biomarkers.) Various forms of mass spectrometry are useful for dectecting the protein forms, including laser desorption approaches, such as traditional MALDI or SELDI, and electrospray ionization.

Thus, when reference is made herein to detecting a particular protein or to measuring the amount of a particular protein, it means detecting and measuring the protein with or without resolving various forms of protein. For example, the step of "β-2 microglobulin" includes measuring β-2 microglobulin by means that do not differentiate between various forms of the protein (e.g., certain immunoassays) as well as by means that differentiate some forms from other forms or that measure a specific form of the protein. In contrast, when it is desired to measure a particular form or forms of a protein, e.g., a particular form of β-2 microglobulin, the particular form (or forms) is specified.

### 4. DETECTION OF BIOMARKERS FOR OVARIAN CANCER

The biomarkers of this invention as defined above can be detected by any suitable method. Detection paradigms that can be employed to this end include optical methods, electrochemical methods (voltametry and amperometry techniques), atomic force microscopy, and radio frequency methods, e.g., multipolar resonance spectroscopy. Illustrative of optical methods, in addition to microscopy, both confocal and non-confocal, are detection of fluorescence, luminescence, chemiluminescence, absorbance, reflectance, transmittance, and birefringence or refractive index (e.g., surface plasmon resonance, ellipsometry, a resonant mirror method, a grating coupler waveguide method or interferometry).

In one embodiment, a sample is analyzed by means of a biochip. Biochips generally comprise solid substrates and have a generally planar surface, to which a capture reagent (also called an adsorbent or affinity reagent) is attached. Frequently, the surface of a biochip comprises a plurality of addressable locations, each of which has the capture reagent bound there.

Protein biochips are biochips adapted for the capture of polypeptides. Many protein biochips are described in the art. These include, for example, protein biochips produced by Ciphergen Biosystems, Inc. (Fremont, CA), Zyomyx (Hayward, CA), Invitrogen (Carlsbad, CA), Biacore (Uppsala, Sweden) and Procognia (Berkshire, UK) . Examples of such protein biochips are described in the following patents or published patent applications: U.S. Patent No. 6,225,047 (Hutchens & Yip); U.S. Patent No. 6,537,749 (Kuimelis and Wagner); U.S. Patent No. 6,329,209 (Wagner et al.); PCT International Publication No. WO 00/56934 (Englert et al.); PCT International Publication No. WO 03/048768 (Boutell et al.) and U.S. Patent No. 5,242,828 (Bergstrom et al.).

### 4.1. Detection by Mass Spectrometry

In a preferred embodiment, the biomarkers of this invention are detected by mass spectrometry, a method that employs a mass spectrometer to detect gas phase ions. Examples of mass spectrometers are time-of-flight, magnetic sector, quadrupole filter, ion trap, ion cyclotron resonance, electrostatic sector analyzer and hybrids of these.

In a further preferred method, the mass spectrometer is a laser desorption/ionization mass spectrometer. In laser desorption/ionization mass spectrometry, the analytes are placed on the surface of a mass spectrometry probe, a device adapted to engage a probe interface of the mass spectrometer and to present an analyte to ionizing energy for ionization and introduction into a mass spectrometer. A laser desorption mass spectrometer employs laser energy, typically from an ultraviolet laser, but also from an infrared laser, to desorb analytes from a surface, to volatilize and ionize them and make them available to the ion optics of the mass spectrometer. The analyis of proteins by LDI can take the form of MALDI or of SELDI. The analyis of proteins by LDI can take the form of MALDI or of SELDI.

Laser desorption/ionization in a single TOF instrument typically is performed in linear extraction mode. Tandem mass spectrometers can employ orthogonal extraction modes.

### 4.1.1. SELDI

A preferred mass spectrometric technique for use in the invention is "Surface Enhanced Laser Desorption and Ionization" or "SELDI," as described, for example, in U.S. Patents No. 5,719,060 and No. 6,225,047, both to Hutchens and Yip. This refers to a method of desorption/ionization gas phase ion spectrometry (e.g., mass spectrometry) in which an analyte (here, one or more of the biomarkers) is captured on the surface of a SELDI mass spectrometry probe.

SELDI also has been called is called "affinity capture mass spectrometry." It also is called "Surface-Enhanced Affinity Capture" or "SEAC". This version involves the use of probes that have a material on the probe surface that captures analytes through a noncovalent affinity interaction (adsorption) between the material and the analyte. The material is variously called an "adsorbent," a "capture reagent," an "affinity reagent" or a "binding moiety." Such probes can be referred to as "affinity capture probes" and as having an "adsorbent surface." The capture reagent can be any material capable of binding an analyte. The capture reagent is attached to the probe surface by physisorption or chemisorption. In certain embodiments the probes have the capture reagent already attached to the surface. In other embodiments, the probes are pre-activated and include a reactive moiety that is capable of binding the capture reagent, e.g., through a reaction forming a covalent or coordinate covalent bond. Epoxide and acyl-imidizole are useful reactive moieties to covalently bind polypeptide capture reagents such as antibodies or cellular receptors. Nitrilotriacetic acid and iminodiacetic acid are useful reactive moieties that function as chelating agents to bind metal ions that interact non-covalently with histidine containing peptides. Adsorbents are generally classified as chromatographic adsorbents and biospecific adsorbents.

"Chromatographic adsorbent" refers to an adsorbent material typically used in chromatography. Chromatographic adsorbents include, for example, ion exchange materials, metal chelators (e.g., nitrilotriacetic acid or iminodiacetic acid), immobilized metal chelates, hydrophobic interaction adsorbents, hydrophilic interaction adsorbents, dyes, simple biomolecules (e.g., nucleotides, amino acids, simple sugars and fatty acids) and mixed mode adsorbents (*e*.*g*., hydrophobic attraction/electrostatic repulsion adsorbents).

"Biospecific adsorbent" refers to an adsorbent comprising a biomolecule, e.g., a nucleic acid molecule (*e*.*g*., an aptamer), a polypeptide, a polysaccharide, a lipid, a steroid or a conjugate of these (*e*.*g*., a glycoprotein, a lipoprotein, a glycolipid, a nucleic acid (*e*.*g*., DNA)-protein conjugate). In certain instances, the biospecific adsorbent can be a macromolecular structure such as a multiprotein complex, a biological membrane or a virus. Examples of biospecific adsorbents are antibodies, receptor proteins and nucleic acids. Biospecific adsorbents typically have higher specificity for a target analyte than chromatographic adsorbents. Further examples of adsorbents for use in SELDI can be found in U.S. Patent No. 6,225,047. A "bioselective adsorbent" refers to an adsorbent that binds to an analyte with an affinity of at least 10⁻⁸ M.

Protein biochips produced by Ciphergen comprise surfaces having chromatographic or biospecific adsorbents attached thereto at addressable locations. Ciphergen's ProteinChip® arrays include NP20 (hydrophilic); H4 and H50 (hydrophobic); SAX-2, Q-10 and (anion exchange); WCX-2 and CM-10 (cation exchange); IMAC-3, IMAC-30 and IMAC-50 (metal chelate);and PS-10, PS-20 (reactive surface with acyl-imidizole, epoxide) and PG-20 (protein G coupled through acyl-imidizole). Hydrophobic ProteinChip arrays have isopropyl or nonylphenoxy-poly(ethylene glycol)methacrylate functionalities. Anion exchange ProteinChip arrays have quaternary ammonium functionalities. Cation exchange ProteinChip arrays have carboxylate functionalities. Immobilized metal chelate ProteinChip arrays have nitrilotriacetic acid functionalities (IMAC 3 and IMAC 30) or O-methacryloyl-N,N-bis-carboxymethyl tyrosine funtionalities (IMAC 50) that adsorb transition metal ions, such as copper, nickel, zinc, and gallium, by chelation. Preactivated ProteinChip arrays have acyl-imidizole or epoxide functional groups that can react with groups on proteins for covalent binding.

Such biochips are further described in: U.S. Patent No. 6,579,719 (Hutchens and Yip, "Retentate Chromatography," June 17, 2003); U.S. Patent 6,897,072 (Rich et al., "Probes for a Gas Phase Ion Spectrometer," May 24, 2005); U.S. Patent No. 6,555,813 (Beecher et al., "Sample Holder with Hydrophobic Coating for Gas Phase Mass Spectrometer," April 29, 2003); U.S. Patent Publication No. U.S. 2003 -0032043 A1 (Pohl and Papanu, "Latex Based Adsorbent Chip," July 16, 2002); and PCT International Publication No. WO 03/040700 (Um et al., "Hydrophobic Surface Chip," May 15, 2003); U.S. Patent ApplicationPublication No. US 2003/-0218130 A1 (Boschetti et al., "Biochips With Surfaces Coated With Polysaccharide-Based Hydrogels," April 14, 2003) and U.S. Patent 7,045,366 (Huang et al., "Photocrosslinked Hydrogel Blend Surface Coatings" May 16, 2006).

In general, a probe with an adsorbent surface is contacted with the sample for a period of time sufficient to allow the biomarker or biomarkers that may be present in the sample to bind to the adsorbent. After an incubation period, the substrate is washed to remove unbound material. Any suitable washing solutions can be used; preferably, aqueous solutions are employed. The extent to which molecules remain bound can be manipulated by adjusting the stringency of the wash. The elution characteristics of a wash solution can depend, for example, on pH, ionic strength, hydrophobicity, degree of chaotropism, detergent strength, and temperature. Unless the probe has both SEAC and SEND properties (as described herein), an energy absorbing molecule then is applied to the substrate with the bound biomarkers.

In yet another method, one can capture the biomarkers with a solid-phase bound immuno-adsorbent that has antibodies that bind the biomarkers. After washing the adsorbent to remove unbound material, the biomarkers are eluted from the solid phase and detected by applying to a SELDI biochip that binds the biomarkers and analyzing by SELDI.

The biomarkers bound to the substrates are detected in a gas phase ion spectrometer such as a time-of-flight mass spectrometer. The biomarkers are ionized by an ionization source such as a laser, the generated ions are collected by an ion optic assembly, and then a mass analyzer disperses and analyzes the passing ions. The detector then translates information of the detected ions into mass-to-charge ratios. Detection of a biomarker typically will involve detection of signal intensity. Thus, both the quantity and mass of the biomarker can be determined.

### 4.1.2. SEND

Another method of laser desorption mass spectrometry is called Surface-Enhanced Neat Desorption ("SEND"). SEND involves the use of probes comprising energy absorbing molecules that are chemically bound to the probe surface ("SEND probe"). The phrase "energy absorbing molecules" (EAM) denotes molecules that are capable of absorbing energy from a laser desorption/ionization source and, thereafter, contribute to desorption and ionization of analyte molecules in contact therewith. The EAM category includes molecules used in MALDI, frequently referred to as "matrix," and is exemplified by cinnamic acid derivatives, sinapinic acid (SPA), cyano-hydroxy-cinnamic acid (CHCA) and dihydroxybenzoic acid, ferulic acid, and hydroxyaceto-phenone derivatives. In certain embodiments, the energy absorbing molecule is incorporated into a linear or cross-linked polymer, *e*.*g*., a polymethacrylate. For example, the composition can be a co-polymer of α-cyano-4-methacryloyloxycinnamic acid and acrylate. In another embodiment, the composition is a co-polymer of α-cyano-4-methacryloyloxycinnamic acid, acrylate and 3-(tri-ethoxy)silyl propyl methacrylate. In another embodiment, the composition is a co-polymer of α-cyano-4-methacryloyloxycinnamic acid and octadecylmethacrylate ("C18 SEND"). SEND is further described in U.S. Patent No. 6,124,137 and PCT International Publication No. WO 03/64594 (Kitagawa, "Monomers And Polymers Having Energy Absorbing Moieties Of Use In Desorption/Ionization Of Analytes," August 7, 2003).

SEAC/SEND is a version of laser desorption mass spectrometry in which both a capture reagent and an energy absorbing molecule are attached to the sample presenting surface. SEAC/SEND probes therefore allow the capture of analytes through affinity capture and ionization/desorption without the need to apply external matrix. The C18 SEND biochip is a version of SEAC/SEND, comprising a C18 moiety which functions as a capture reagent, and a CHCA moiety which functions as an energy absorbing moiety.

### 4.1.3. SEPAR

Another version of LDI, called Surface-Enhanced Photolabile Attachment and Release ("SEPAR"). SEPAR involves the use of probes having moieties attached to the surface that can covalently bind an analyte, and then release the analyte through breaking a photolabile bond in the moiety after exposure to light, *e*.*g*., to laser light (*see*, U.S. Patent No. 5,719,060). SEPAR and other forms of SELDI are readily adapted to detecting a biomarker or biomarker profile, pursuant to the present invention.

### 4.1.4. MALDI

MALDI is a traditional method of laser desorption/ionization used to analyte biomolecules such as proteins and nucleic acids. In one MALDI method, the sample is mixed with matrix and deposited directly on a MALDI array. However, the complexity of biological samples such as serum and urine makes this method less than optimal without prior fractionation of the sample. Accordingly, in certain embodiments with biomarkers are preferably first captured with biospecific (e.g., an antibody) or chromatographic materials coupled to a solid support such as a resin (e.g., in a spin column). Specific affinity materials that bind the biomarkers of this invention are described above. After purification on the affinity material, the biomarkers are eluted and then detected by MALDI.

### 4.1.5.

In another mass spectrometry method, the biomarkers can be first captured on a chromatographic resin having chromatographic properties that bind the biomarkers. In the present example, this could include a variety of methods. For example, one could capture the biomarkers on a cation exchange resin, such as CM Ceramic HyperD F resin, wash the resin, elute the biomarkers and detect by MALDI. Alternatively, this method could be preceded by fractionating the sample on an anion exchange resin before application to the cation exchange resin. In another alternative, one could fractionate on an anion exchange resin and detect by MALDI directly. In yet another method, one could capture the biomarkers on an immuno-chromatographic resin that comprises antibodies that bind the biomarkers, wash the resin to remove unbound material, elute the biomarkers from the resin and detect the eluted biomarkers by MALDI or by SELDI.

### 4.1.6. Other forms of ionization in mass spectrometry

In another method, the biomarkers are detected by LC-MS or LC-LC-MS. This involves resolving the proteins in a sample by one or two passes through liquid chromatography, followed by mass spectrometry analysis, typically electrospray ionization.

### 4.1.7. Data Analysis

Analysis of analytes by time-of-flight mass spectrometry generates a time-of-flight spectrum. The time-of-flight spectrum ultimately analyzed typically does not represent the signal from a single pulse of ionizing energy against a sample, but rather the sum of signals from a number of pulses. This reduces noise and increases dynamic range. This time-of-flight data is then subject to data processing. In Ciphergen's ProteinChip® software, data processing typically includes TOF-to-M/Z transformation to generate a mass spectrum, baseline subtraction to eliminate instrument offsets and high frequency noise filtering to reduce high frequency noise.

Data generated by desorption and detection of biomarkers can be analyzed with the use of a programmable digital computer. The computer program analyzes the data to indicate the number of biomarkers detected, and optionally the strength of the signal and the determined molecular mass for each biomarker detected. Data analysis can include steps of determining signal strength of a biomarker and removing data deviating from a predetermined statistical distribution. For example, the observed peaks can be normalized, by calculating the height of each peak relative to some reference.

The computer can transform the resulting data into various formats for display. The standard spectrum can be displayed, but in one useful format only the peak height and mass information are retained from the spectrum view, yielding a cleaner image and enabling biomarkers with nearly identical molecular weights to be more easily seen. In another useful format, two or more spectra are compared, conveniently highlighting unique biomarkers and biomarkers that are up- or down-regulated between samples. Using any of these formats, one can readily determine whether a particular biomarker is present in a sample.

Analysis generally involves the identification of peaks in the spectrum that represent signal from an analyte. Peak selection can be done visually, but software is available, as part of Ciphergen's ProteinChip® software package, that can automate the detection of peaks. In general, this software functions by identifying signals having a signal-to-noise ratio above a selected threshold and labeling the mass of the peak at the centroid of the peak signal. In one useful application, many spectra are compared to identify identical peaks present in some selected percentage of the mass spectra. One version of this software clusters all peaks appearing in the various spectra within a defined mass range, and assigns a mass (M/Z) to all the peaks that are near the mid-point of the mass (M/Z) cluster.

Software used to analyze the data can include code that applies an algorithm to the analysis of the signal to determine whether the signal represents a peak in a signal that corresponds to a biomarker according to the present invention. The software also can subject the data regarding observed biomarker peaks to classification tree or ANN analysis, to determine whether a biomarker peak or combination of biomarker peaks is present that indicates the status of the particular clinical parameter under examination. Analysis of the data may be "keyed" to a variety of parameters that are obtained, either directly or indirectly, from the mass spectrometric analysis of the sample. These parameters include, but are not limited to, the presence or absence of one or more peaks, the shape of a peak or group of peaks, the height of one or more peaks, the log of the height of one or more peaks, and other arithmetic manipulations of peak height data.

### 4.1.8. General protocol for SELDI detection of biomarkers for ovarian cancer

A preferred protocol for the detection of the biomarkers of this invention is as follows. The biological sample to be tested, e.g., serum, preferably is subject to pre-fractionation before SELDI analysis. This simplifies the sample and improves sensitivity. A preferred method of pre-fractionation involves contacting the sample with an anion exchange chromatographic material, such as Q HyperD (BioSepra, SA). The bound materials are then subject to stepwise pH elution using buffers at pH 9, pH 7, pH 5 and pH 4. (See Example 1 - Buffer list.) Various fractions containing the biomarker are collected.

The sample to be tested (preferably pre-fractionated) is then contacted with an affinity capture probe comprising an cation exchange adsorbent (preferably a WCX ProteinChip array (Ciphergen Biosystems, Inc.)) or an IMAC adsorbent (preferably an IMAC3 ProteinChip array (Ciphergen Biosystems, Inc.)), again as indicated in Table 1. The probe is washed with a buffer that will retain the biomarker while washing away unbound molecules. A suitable wash for each biomarker is the buffer identified in the Examples. The biomarkers are detected by laser desorption/ionization mass spectrometry.

Alternatively, if antibodies that recognize the biomarker are available, for example in the case of PF4, β2-microglobulin, vitamin D binding protein, or albumin, these can be attached to the surface of a probe, such as a pre-activated PS10 or PS20 ProteinChip array (Ciphergen Biosystems, Inc.). These antibodies can capture the biomarkers from a sample onto the probe surface. Then the biomarkers can be detected by, e.g., laser desorption/ionization mass spectrometry.

### 4.2 Detection by Immunoassay

In another embodiment of the invention, the biomarkers of the invention are measured by a method other than mass spectrometry or other than methods that rely on a measurement of the mass of the biomarker. In one such embodiment that does not rely on mass, the biomarkers of this invention are measured by immunoassay. Immunoassay requires biospecific capture reagents, such as antibodies, to capture the biomarkers. Antibodies can be produced by methods well known in the art, *e*.*g*., by immunizing animals with the biomarkers. Biomarkers can be isolated from samples based on their binding characteristics. Alternatively, if the amino acid sequence of a polypeptide biomarker is known, the polypeptide can be synthesized and used to generate antibodies by methods well known in the art.

This invention contemplates traditional immunoassays including, for example, sandwich immunoassays including ELISA or fluorescence-based immunoassays, as well as other enzyme immunoassays. Nephelometry is an assay done in liquid phase, in which antibodies are in solution. Binding of the antigen to the antibody results in changes in absorbance, which is measured. In the SELDI-based immunoassay, a biospecific capture reagent for the biomarker is attached to the surface of an MS probe, such as a pre-activated ProteinChip array. The biomarker is then specifically captured on the biochip through this reagent, and the captured biomarker is detected by mass spectrometry.

### 5. DETERMINATION OF SUBJECT OVARIAN CANCER STATUS

The biomarkers of the invention as defined above are used in diagnostic tests to assess ovarian cancer status in a subject, *i.e.* to diagnose ovarian cancer. The phrase "ovarian cancer status" includes any distinguishable manifestation of the disease, including non-disease. For example, ovarian cancer status includes, without limitation, the presence or absence of disease (e.g., ovarian cancer v. non-ovarian cancer), the risk of developing disease, the stage of the disease, the progression of disease (e.g., progress of disease or remission of disease over time) and the effectiveness or response to treatment of disease.

The correlation of test results with ovarian cancer status involves applying a classification algorithm of some kind to the results to generate the status. The classification algorithm may be as simple as determining whether or not the amount of a marker as defined above, e.g., β-2 microglobulin, measured is above or below a particular cut-off number. When multiple biomarkers are used, the classification algorithm may be a linear regression formula. Alternatively, the classification algorithm may be the product of any of a number of learning algorithms described herein.

In the case of complex classification algorithms, it may be necessary to perform the algorithm on the data, thereby determining the classification, using a computer, e.g., a programmable ditigal computer. In either case, one can then record the status on tangible medium, for example, in computer-readable format such as a memory drive or disk or simply printed on paper. The result also could be reported on a computer screen.

### 5.1.1 Single Markers

The biomarkers of the invention as defined above are used in diagnostic tests to assess ovarian cancer status in a subject, *i.e.* to diagnose ovarian cancer. The phrase "ovarian cancer status" includes any distinguishable manifestation of the disease, including non-disease. For example, disease status includes, without limitation, the presence or absence of disease (e.g., ovarian cancer v. non-ovarian cancer), the risk of developing disease, the stage of the disease, the progress of disease (e.g., progress of disease or remission of disease over time) and the effectiveness or response to treatment of disease. Based on this status, further procedures may be indicated, including additional diagnostic tests or therapeutic procedures or regimens.

The power of a diagnostic test to correctly predict status is commonly measured as the sensitivity of the assay, the specificity of the assay or the area under a receiver operated characteristic ("ROC") curve. Sensitivity is the percentage of true positives that are predicted by a test to be positive, while specificity is the percentage of true negatives that are predicted by a test to be negative. An ROC curve provides the sensitivity of a test as a function of 1-specificity. The greater the area under the ROC curve, the more powerful the predictive value of the test. Other useful measures of the utility of a test are positive predictive value and negative predictive value. Positive predictive value is the percentage of people who test positive that are actually positive. Negative predictive value is the percentage of people who test negative that are actually negative.

The biomarkers of this invention show a statistical difference in different ovarian cancer statuses of at least p ≤ 0.05, p ≤ 10⁻², p ≤ 10⁻³, p ≤ 10⁻⁴ or p ≤ 10⁻⁵. Diagnostic tests that use these biomarkers alone or in combination show a sensitivity and specificity of at least 75%, at least 80%, at least 85%, at least 90%, at least 95%, at least 98% and about 100%.

Each biomarker listed in Table 1 is differentially present in ovarian cancer, and, therefore, each is individually useful in aiding in the determination of ovarian cancer status. The method involves, first, measuring the selected biomarker in a subject sample using the methods described herein, e.g., capture on a SELDI biochip followed by detection by mass spectrometry and, second, comparing the measurement with a diagnostic amount or cut-off that distinguishes a positive ovarian cancer status from a negative ovarian cancer status. The diagnostic amount represents a measured amount of a biomarker above which or below which a subject is classified as having a particular ovarian cancer status. For example, if the biomarker is up-regulated compared to normal during ovarian cancer, then a measured amount above the diagnostic cutoff provides a diagnosis of ovarian cancer. Alternatively, if the biomarker is down-regulated during ovarian cancer, then a measured amount below the diagnostic cutoff provides a diagnosis of ovarian cancer. As is well understood in the art, by adjusting the particular diagnostic cut-off used in an assay, one can increase sensitivity or specificity of the diagnostic assay depending on the preference of the diagnostician. The particular diagnostic cut-off can be determined, for example, by measuring the amount of the biomarker in a statistically significant number of samples from subjects with the different ovarian cancer statuses, as was done here, and drawing the cut-off to suit the diagnostician's desired levels of specificity and sensitivity.

### 5.2. Combinations of Markers

While individual biomarkers are useful diagnostic biomarkers, it has been found that a combination of biomarkers can provide greater predictive value of a particular status than asingle biomarker alone. Specifically, the detection of a plurality of biomarkers in a sample can increase the sensitivity and/or specificity of the test. A combination of at least two biomarkers, preferably at least three or more than three biomarkers, is sometimes referred to as a "biomarker profile" or "biomarker fingerprint." Accordingly, CTAP3 can be combined with other biomarkers for ovarian or endometrial cancer to improve the sensitivity and/or specificity of the diagnostic test.

In particular, a diagnostic test for ovarian cancer status involving the measurement of a biomarker listed in Table 1, e.g., β-2 microglobulin, and any of the following biomarkers for ovarian cancer identified in Table 3 (including their modified forms where appropriate) can have greater predictive power than the measurement of a biomarker identified in Table 1, e.g., β-2 microglobulin, alone:

| Table 3 | |
|---|---|
| Marker | Comments (up- or down-regulated in cancer) |
| Transferrin | Down-regulated; 79 kD, detected on IMAC ProteinChip array charged with nickel |
| | WO 03/057014 |
| Haptoglobin precursor protein fragment | Up-regulated; 9.2 kD detected on IMAC ProteinChip array charged with nickel |
| | WO 03/057014 |
| ApoA1 | Down-regulated; predicted mass 28078.62D; detected on IMAC or H50 ProteinChip array. |
| | WO 2004/013609 |
| Transthyretin and transthyretin delta N 10 | Down-regulated; predicted mass 13761D and 12887 D, respectively; detected on Q10 ProteinChip array. |
| | WO 2004/013609 |
| ITIH4 internal fragments | Up-regulated; among other fragments: MNFRPGVLSSRQLGLPGPPDVPDHAAYHPF (SEQ ID NO: 1), a fragment spanning amino acids 660-689 of human Inter-alpha trypsin inhibitor, heavy chain H4, predicted mass: 3273.72 D; detected on IMAC ProteinChip array WO 2004/013609 and WO 2005/098447 |
| CTAP3 | Up-regulated; detected at 9313.9D on IMAC-Cu ProteinChip array US Provisional Application 60/693,324, filed June 22, 2005; US Application 11/XXX,XXX, filed June 21, 2006, entitled "BIOMARKER FOR OVARIAN CANCER: CTAP3-RELATED PROTEINS" |
| Hepcidin and modified forms | Up-regulated; detected by SELDI - coprecipitate with ITIH4 fragment. |
| | Hepcidin-25 (SEQ ID NO: 2): DTHFPICIFCCGCCHRSKCGMCCKT |
| | Hepcidin-24 (SEQ ID NO: 3): THFPICIFCCGCCHRSKCGMCCKT |
| | Hepcidin-22 (SEQ ID NO: 3): FPICIFCCGCCHRSKCGM CCKT |
| | Hepcidin-20 (SEQ ID NO: 4): ICIFCCGCCHRSKCGMCCKT |
| Haptoglobin alpha | Up-regulated. Detected at 11,600D-11,700D on an IMAC ProteinChip array charged with copper; |
| | WO 02/100242 |
| Prostatin | Up-regulated |
| | USP 6,846,642 |
| Osteopontin | Up-regulated In urine - GlycosylatedUS 2005-0009120 A1 |
| | In serum - US 2005-0214826 |
| Eosinophil-derived neurotoxin | Up regulated in urine. Glycosylated Detected at 17.4 KDa on a WCX2 ProteinChip array. |
| | US 2005-0009120 A1 |
| leptin | Down-regulated; |
| | US 2005-0214826 |
| prolactin | Up-regulated; |
| | US 2005-0214826 |
| IGF-II | Down-regulated; |
| | US 2005-0214826 |
| Hemoglobin (alpha-hemoglobin, beta-hemoglobin) | Up-regulated; |
| | WO 2006-019906 |
| [CA 125 | Up-regulated |

In a study on samples of a Japanese cohort, the combination of hepcidin, ApoAl, β2 microglobulin and CTAP-III was found to be a particularly effective diagnostic combination.

Other biomarkers with which one or more biomarkers identified in Table 1 can be combined include, but are not limited to, CA125 II, CA15-3, CA19-9, CA72-4, CA 195, tumor associated trypsin inhibitor (TATI), CEA, placental alkaline phosphatase (PLAP), Sialyl TN, galactosyltransferase, macrophage colony stimulating factor (M-CSF, CSF-1), lysophosphatidic acid (LPA), 110 kD component of the extracellular domain of the epidermal growth factor receptor (p110EGFR), tissue kallikreins, e.g., kallikrein 6 and kallikrein 10 (NES-1), prostasin, HE4, creatine kinase B (CKB), LASA, HER-2/neu, urinary gonadotropin peptide, Dianon NB 70/K, Tissue peptide antigen (TPA), SMRP, osteopontin, and haptoglobin, leptin, prolactin, insulin like growth factor I or II.

### 5.3 Determining Risk of Developing Disease

Further described are methods for determining the risk of developing disease in a subject. Biomarker amounts or patterns are characteristic of various risk states, e.g., high, medium or low. The risk of developing a disease is determined by measuring the relevant biomarker or biomarkers and then either submitting them to a classification algorithm or comparing them with a reference amount and/or pattern of biomarkers that is associated with the particular risk level.

### 5.4 Determining Stage of Disease

Further disclosed are methods for determining the stage of disease in a subject. Each stage of the disease has a characteristic amount of a biomarker or relative amounts of a set of biomarkers (a pattern). The stage of a disease is determined by measuring the relevant biomarker or biomarkers and then either submitting them to a classification algorithm or comparing them with a reference amount and/or pattern of biomarkers that is associated with the particular stage.

### 5.5 Determining Course (Progression/Remission) of Disease

Further described are methods for determining the course of disease in a subject. Disease course refers to changes in disease status over time, including disease progression (worsening) and disease regression (improvement). Over time, the amounts or relative amounts (e.g., the pattern) of the biomarkers change. For example, biomarkers M 3886.8 and M 4145.8 are increased with disease, while biomarker M 10515.4 is decreased in disease. Therefore, the trend of these markers, either increased or decreased over time toward diseased or non-diseased indicates the course of the disease. Accordingly, this method involves measuring one or more biomarkers in a subject at at least two different time points, e.g., a first time and a second time, and comparing the change in amounts, if any. The course of disease is determined based on these comparisons.

### 5.6 Reporting the Status

Further described is the communication of assay results or diagnoses or both to technicians, physicians or patients, for example. In certain embodiments, computers will be used to communicate assay results or diagnoses or both to interested parties, e.g., physicians and their patients. In some embodiments, the assays will be performed or the assay results analyzed in a country or jurisdiction which differs from the country or jurisdiction to which the results or diagnoses are communicated.

In a variant, a diagnosis based on the differential presence in a test subject of any the biomarkers of Table 1 is communicated to the subject as soon as possible after the diagnosis is obtained. The diagnosis may be communicated to the subject by the subject's treating physician. Alternatively, the diagnosis may be sent to a test subject by email or communicated to the subject by phone. A computer may be used to communicate the diagnosis by email or phone. In certain variants, the message containing results of a diagnostic test may be generated and delivered automatically to the subject using a combination of computer hardware and software which will be familiar to artisans skilled in telecommunications. One example of a healthcare-oriented communications system is described in U.S. Patent Number 6,283,761; however, the variant is not limited to methods which utilize this particular communications system. In certain embodiments of the methods, all or some of the method steps, including the assaying of samples, diagnosing of diseases, and communicating of assay results or diagnoses, may be carried out in diverse (e.g., foreign) jurisdictions.

### 5.7 Subject Management

Further described are methods of qualifying ovarian cancer status, the methods further comprising managing subject treatment based on the status. Such management includes the actions of the physician or clinician subsequent to determining ovarian cancer status. For example, if a physician makes a diagnosis of ovarian cancer, then a certain regime of treatment, such as prescription or administration of therapeutic agent might follow. Alternatively, a diagnosis of non-ovarian cancer or non-ovarian cancer might be followed with further testing to determine a specific disease that might the patient might be suffering from. Also, if the diagnostic test gives an inconclusive result on ovarian cancer status, further tests may be called for.

Additional variants relate to the communication of assay results or diagnoses or both to technicians, physicians or patients, for example. In certain variants, computers will be used to communicate assay results or diagnoses or both to interested parties, e.g., physicians and their patients. In some variants, the assays will be performed or the assay results analyzed in a country or jurisdiction which differs from the country or jurisdiction to which the results or diagnoses are communicated.

In a variant, a diagnosis based on the presence or absence in a test subject of any the biomarkers of Table 1 is communicated to the subject as soon as possible after the diagnosis is obtained. The diagnosis may be communicated to the subject by the subject's treating physician. Alternatively, the diagnosis may be sent to a test subject by email or communicated to the subject by phone. A computer may be used to communicate the diagnosis by email or phone. In certain variants, the message containing results of a diagnostic test may be generated and delivered automatically to the subject using a combination of computer hardware and software which will be familiar to artisans skilled in telecommunications. One example of a healthcare-oriented communications system is described in U.S. Patent Number 6,283,761; however, the variant is not limited to methods which utilize this particular communications system. In certain embodiments of the methods, all or some of the method steps, including the assaying of samples, diagnosing of diseases, and communicating of assay results or diagnoses, may be carried out in diverse (e.g., foreign) jurisdictions.

### 5.8 Determining Therapeutic Efficacy of Pharmaceutical Drug

Further described are methods for determining the therapeutic efficacy of a pharmaceutical drug. These methods are useful in performing clinical trials of the drug, as well as monitoring the progress of a patient on the drug. Therapy or clinical trials involve administering the drug in a particular regimen. The regimen may involve a single dose of the drug or multiple doses of the drug over time. The doctor or clinical researcher monitors the effect of the drug on the patient or subject over the course of administration. If the drug has a pharmacological impact on the condition, the amounts or relative amounts (e.g., the pattern or profile) of the biomarkers of this invention changes toward a non-disease profile. For example, biomarkers M 3886.8 and M 4145.8 are increased with disease, while biomarker M 10515.4 is decreased in disease. Therefore, one can follow the course of the amounts of these biomarkers in the subject during the course of treatment. Accordingly, this method involves measuring one or more biomarkers in a subject receiving drug therapy, and correlating the amounts of the biomarkers with the disease status of the subject. One variant of this method involves determining the levels of the biomarkers at least two different time points during a course of drug therapy, e.g., a first time and a second time, and comparing the change in amounts of the biomarkers, if any. For example, the biomarkers can be measured before and after drug administration or at two different time points during drug administration. The effect of therapy is determined based on these comparisons. If a treatment is effective, then the biomarkers will trend toward normal, while if treatment is ineffective, the biomarkers will trend toward disease indications. If a treatment is effective, then the biomarkers will trend toward normal, while if treatment is ineffective, the biomarkers will trend toward disease indications.

### 6. GENERATION OF CLASSIFICATION ALGORITHMS FOR QUALIFYING OVARIAN CANCER STATUS

In some variants, data derived from the spectra (e.g., mass spectra or time-of-flight spectra) that are generated using samples such as "known samples" can then be used to "train" a classification model. A "known sample" is a sample that has been pre-classified. The data that are derived from the spectra and are used to form the classification model can be referred to as a "training data set." Once trained, the classification model can recognize patterns in data derived from spectra generated using unknown samples. The classification model can then be used to classify the unknown samples into classes. This can be useful, for example, in predicting whether or not a particular biological sample is associated with a certain biological condition (e.g., diseased versus non-diseased).

The training data set that is used to form the classification model may comprise raw data or pre-processed data. In some variants, raw data can be obtained directly from time-of-flight spectra or mass spectra, and then may be optionally "pre-processed" as described above.

Classification models can be formed using any suitable statistical classification (or "learning") method that attempts to segregate bodies of data into classes based on objective parameters present in the data. Classification methods may be either supervised or unsupervised. Examples of supervised and unsupervised classification processes are described in Jain, "Statistical Pattern Recognition: A Review", IEEE Transactions on Pattern Analysis and Machine Intelligence, Vol. 22, No. 1, January 2000.

In supervised classification, training data containing examples of known categories are presented to a learning mechanism, which learns one or more sets of relationships that define each of the known classes. New data may then be applied to the learning mechanism, which then classifies the new data using the learned relationships. Examples of supervised classification processes include linear regression processes (*e*.*g*., multiple linear regression (MLR), partial least squares (PLS) regression and principal components regression (PCR)), binary decision trees (e.g., recursive partitioning processes such as CART - classification and regression trees), artificial neural networks such as back propagation networks, discriminant analyses (e.g., Bayesian classifier or Fischer analysis), logistic classifiers, and support vector classifiers (support vector machines).

A preferred supervised classification method is a recursive partitioning process. Recursive partitioning processes use recursive partitioning trees to classify spectra derived from unknown samples. Further details about recursive partitioning processes are provided in U.S. Patent Application No. 2002 0138208 A1 to Paulse et al., "Method for analyzing mass spectra."

In other variants, the classification models that are created can be formed using unsupervised learning methods. Unsupervised classification attempts to learn classifications based on similarities in the training data set, without pre-classifying the spectra from which the training data set was derived. Unsupervised learning methods include cluster analyses. A cluster analysis attempts to divide the data into "clusters" or groups that ideally should have members that are very similar to each other, and very dissimilar to members of other clusters. Similarity is then measured using some distance metric, which measures the distance between data items, and clusters together data items that are closer to each other. Clustering techniques include the MacQueen's K-means algorithm and the Kohonen's Self-Organizing Map algorithm.

Learning algorithms asserted for use in classifying biological information are described, for example, in PCT International Publication No. WO O1/31580 (Barnhill et al., "Methods and devices for identifying patterns in biological systems and methods of use thereof'), U.S. Patent Application No. 2002 0193950 A1 (Gavin et al., "Method or analyzing mass spectra"), U.S. Patent Application No. 2003 0004402 A1 (Hitt et al., "Process for discriminating between biological states based on hidden patterns from biological data"), and U.S. Patent Application No. 2003 0055615 A1 (Zhang and Zhang, "Systems and methods for processing biological expression data").

The classification models can be formed on and used on any suitable digital computer. Suitable digital computers include micro, mini, or large computers using any standard or specialized operating system, such as a Unix, Windows™ or Linux™ based operating system. The digital computer that is used may be physically separate from the mass spectrometer that is used to create the spectra of interest, or it may be coupled to the mass spectrometer.

The training data set and the classification models according to embodiments of the invention can be embodied by computer code that is executed or used by a digital computer. The computer code can be stored on any suitable computer readable media including optical or magnetic disks, sticks, tapes, etc., and can be written in any suitable computer programming language including C, C++, visual basic, etc.

The learning algorithms described above are useful both for developing classification algorithms for the biomarkers already discovered, or for finding new biomarkers for ovarian cancer. The classification algorithms, in turn, form the base for diagnostic tests by providing diagnostic values (e.g., cut-off points) for biomarkers used singly or in combination.

### 7. COMPOSITIONS OF MATTER

Further described are compositions of matter based on the biomarkers of this invention.

In one variant, this invention provides biomarkers of this invention in purified form. Purified biomarkers have utility as antigens to raise antibodies. Purified biomarkers also have utility as standards in assay procedures. As used herein, a "purified biomarker" is a biomarker that has been isolated from other proteins and peptides, and/or other material from the biological sample in which the biomarker is found. Biomarkers may be purified using any method known in the art, including, but not limited to, mechanical separation (e.g., centrifugation), ammonium sulphate precipitation, dialysis (including size-exclusion dialysis), size-exclusion chromatography, affinity chromatography, anion-exchange chromatography, cation-exchange chromatography, and methal-chelate chromatography. Such methods may be performed at any appropriate scale, for example, in a chromatography column, or on a biochip.

In another variant there is described a biospecific capture reagent, optionally in purified form, that specifically binds a biomarker of this invention. In one variant, the biospecific capture reagent is an antibody. Such compositions are useful for detecting the biomarker in a detection assay, e.g., for diagnostics.

In another variant there is described an article comprising a biospecific capture reagent that binds a biomarker of this invention, wherein the reagent is bound to a solid phase. For example, this variant contemplates a device comprising bead, chip, membrane, monolith or microtiter plate derivatized with the biospecific capture reagent. Such articles are useful in biomarker detection assays.

In another aspect there is described a composition comprising a biospecific capture reagent, such as an antibody, bound to a biomarker of this invention, the composition optionally being in purified form. Such compositions are useful for purifying the biomarker or in assays for detecting the biomarker.

In another variant there is described an article comprising a solid substrate to which is attached an adsorbent, e.g., a chromatographic adsorbent or a biospecific capture reagent, to which is further bound a biomarker of this invention. In one variant, the article is a biochip or a probe for mass spectrometry, e.g., a SELDI probe. Such articles are useful for purifying the biomarker or detecting the biomarker.

### 8. KITS FOR DETECTION OF BIOMARKERS FOR OVARIAN CANCER

Further described are kits for qualifying ovarian cancer status, which kits are used to detect biomarkers according to the invention. In one variant, the kit comprises a solid support, such as a chip, a microtiter plate or a bead or resin having a capture reagent attached thereon, wherein the capture reagent binds a biomarker of the invention. Thus, for example, the kits can comprise mass spectrometry probes for SELDI, such as ProteinChip® arrays. In the case of biospecfic capture reagents, the kit can comprise a solid support with a reactive surface, and a container comprising the biospecific capture reagent.

The kit can also comprise a washing solution or instructions for making a washing solution, in which the combination of the capture reagent and the washing solution allows capture of the biomarker or biomarkers on the solid support for subsequent detection by, e.g., mass spectrometry. The kit may include more than type of adsorbent, each present on a different solid support.

In a further variant, such a kit can comprise instructions for suitable operational parameters in the form of a label or separate insert. For example, the instructions may inform a consumer about how to collect the sample, how to wash the probe or the particular biomarkers to be detected.

In yet another variant, the kit can comprise one or more containers with biomarker samples, to be used as standard(s) for calibration.

### 9. DETERMINING THERAPEUTIC EFFICACY OF PHARMACEUTICAL DRUG

Further described are methods for determining the therapeutic efficacy of a pharmaceutical drug. These methods are useful in performing clinical trials of the drug, as well as monitoring the progress of a patient on the drug. Therapy or clinical trials involve administering the drug in a particular regimen. The regimen may involve a single dose of the drug or multiple doses of the drug over time. The doctor or clinical researcher monitors the effect of the drug on the patient or subject over the course of administration. If the drug has a pharmacological impact on the condition, the amounts or relative amounts (e.g., the pattern or profile) of the biomarkers of this invention changes toward a non-disease profile. For example, hepcidin is increased with disease, while transthyretin is decreased in disease. Therefore, one can follow the course of the amounts of these biomarkers in the subject during the course of treatment. Accordingly, this method involves measuring one or more biomarkers in a subject receiving drug therapy, and correlating the amounts of the biomarkers with the disease status of the subject. One embodiment of this method involves determining the levels of the biomarkers for at least two different time points during a course of drug therapy, e.g., a first time and a second time, and comparing the change in amounts of the biomarkers, if any. For example, the biomarkers can be measured before and after drug administration or at two different time points during drug administration. The effect of therapy is determined based on these comparisons. If a treatment is effective, then the biomarkers will trend toward normal, while if treatment is ineffective, the biomarkers will trend toward disease indications. If a treatment is effective, then the biomarkers will trend toward normal, while if treatment is ineffective, the biomarkers will trend toward disease indications.

### 10. USE OF BIOMARKERS FOR OVARIAN CANCER IN SCREENING ASSAYS AND METHODS OF TREATING OVARIAN CANCER

The methods of the present invention have other applications as well. For example, the biomarkers can be used to screen for compounds that modulate the expression of the biomarkers *in vitro* or *in vivo,* which compounds in turn may be useful in treating or preventing ovarian cancer in patients. In another example, the biomarkers can be used to monitor the response to treatments for ovarian cancer. In yet another example, the biomarkers can be used in heredity studies to determine if the subject is at risk for developing ovarian cancer.

Thus, for example, the kits could include a solid substrate having a hydrophobic function, such as a protein biochip (*e*.*g*., a Ciphergen H50 ProteinChip array, e.g., ProteinChip array) and a sodium acetate buffer for washing the substrate, as well as instructions providing a protocol to measure the biomarkers of this invention on the chip and to use these measurements to diagnose ovarian cancer.

Compounds suitable for therapeutic testing may be screened initially by identifying compounds which interact with one or more biomarkers listed in Table 1. By way of example, screening might include recombinantly expressing a biomarker listed in Table 1, purifying the biomarker, and affixing the biomarker to a substrate. Test compounds would then be contacted with the substrate, typically in aqueous conditions, and interactions between the test compound and the biomarker are measured, for example, by measuring elution rates as a function of salt concentration. Certain proteins may recognize and cleave one or more biomarkers of Table 1, in which case the proteins may be detected by monitoring the digestion of one or more biomarkers in a standard assay, e.g., by gel electrophoresis of the proteins.

In a related variant, the ability of a test compound to inhibit the activity of one or more of the biomarkers of Table 1 may be measured. One of skill in the art will recognize that the techniques used to measure the activity of a particular biomarker will vary depending on the function and properties of the biomarker. For example, an enzymatic activity of a biomarker may be assayed provided that an appropriate substrate is available and provided that the concentration of the substrate or the appearance of the reaction product is readily measurable. The ability of potentially therapeutic test compounds to inhibit or enhance the activity of a given biomarker may be determined by measuring the rates of catalysis in the presence or absence of the test compounds. The ability of a test compound to interfere with a non-enzymatic (e.g., structural) function or activity of one of the biomarkers of Table 1 may also be measured. For example, the self-assembly of a multi-protein complex which includes one of the biomarkers of Table 1 may be monitored by spectroscopy in the presence or absence of a test compound. Alternatively, if the biomarker is a non-enzymatic enhancer of transcription, test compounds which interfere with the ability of the biomarker to enhance transcription may be identified by measuring the levels of biomarker-dependent transcription *in vivo* or *in vitro* in the presence and absence of the test compound.

Test compounds capable of modulating the activity of any of the biomarkers of Table 1 may be administered to patients who are suffering from or are at risk of developing ovarian cancer or other cancer. For example, the administration of a test compound which increases the activity of a particular biomarker may decrease the risk of ovarian cancer in a patient if the activity of the particular biomarker *in vivo* prevents the accumulation of proteins for ovarian cancer. Conversely, the administration of a test compound which decreases the activity of a particular biomarker may decrease the risk of ovarian cancer in a patient if the increased activity of the biomarker is responsible, at least in part, for the onset of ovarian cancer.

Further described is a method for identifying compounds useful for the treatment of disorders such as ovarian cancer which are associated with increased or decreased levels of modified forms of one or more biomarkers of Table 1.
For example, in one embodiment, cell extracts or expression libraries may be screened for compounds which catalyze the cleavage of full-length M 3886.8 to form truncated forms of M 3886.8. In one embodiment of such a screening assay, cleavage of M 3886.8 may be detected by attaching a fluorophore to M 3886.8 which remains quenched when M 3886.8 is uncleaved but which fluoresces when the protein is cleaved. Alternatively, a version of full-length M 3886.8 modified so as to render the amide bond between amino acids x and y uncleavable may be used to selectively bind or "trap" the cellular protesase which cleaves full-length M 3886.8 at that site *in vivo.* Methods for screening and identifying proteases and their targets are well-documented in the scientific literature, e.g., in Lopez-Ottin et al. (Nature Reviews, 3:509-519 (2002)).

Further described is a method for treating or reducing the progression or likelihood of a disease, e.g., ovarian cancer, which is associated with the increased levels of truncated M 3886.8. For example, after one or more proteins have been identified which cleave full-length M 3886.8, combinatorial libraries may be screened for compounds which inhibit the cleavage activity of the identified proteins. Methods of screening chemical libraries for such compounds are well-known in art. *See*, *e*.*g*., Lopez-Otin et al. (2002). Alternatively, inhibitory compounds may be intelligently designed based on the structure of M 3886.8.

At the clinical level, screening a test compound includes obtaining samples from test subjects before and after the subjects have been exposed to a test compound. The levels in the samples of one or more of the biomarkers listed in Table 1 may be measured and analyzed to determine whether the levels of the biomarkers change after exposure to a test compound. The samples may be analyzed by mass spectrometry, as described herein, or the samples may be analyzed by any appropriate means known to one of skill in the art. For example, the levels of one or more of the biomarkers listed in Table 1 may be measured directly by Western blot using radio- or fluorescently-labeled antibodies which specifically bind to the biomarkers. Alternatively, changes in the levels of mRNA encoding the one or more biomarkers may be measured and correlated with the administration of a given test compound to a subject. In a further embodiment, the changes in the level of expression of one or more of the biomarkers may be measured using *in vitro* methods and materials. For example, human tissue cultured cells which express, or are capable of expressing, one or more of the biomarkers of Table 1 may be contacted with test compounds. Subjects who have been treated with test compounds will be routinely examined for any physiological effects which may result from the treatment. In particular, the test compounds will be evaluated for their ability to decrease disease likelihood in a subject. Alternatively, if the test compounds are administered to subjects who have previously been diagnosed with ovarian cancer, test compounds will be screened for their ability to slow or stop the progression of the disease.

### 11. EXAMPLES

### 11.1. Example 1. Discovery of a biomarker for Ovarian Cancer

### Example 1

Methods: A total of 607 serum samples from five sites were analyzed using SELDI TOF-MS protocols optimized for the seven biomarkers. They included 234 women with benign gynecologic diseases, and 373 patients with invasive epithelial ovarian cancer (101 early stage, 231 late stage, and 40 stage unknown). Among them, 165 benigns and 228 cancers had a CA125 available at time of analysis. The median and quartiles of CA125 for benign, early stage, and late or unknown stage were 26/11/57 IU, 80/22/434 IU, and 234/40/1114 IU, respectively. The biomarkers were assessed individually using the Mann-Whitney U Test. A linear composite index was derived in an unsupervised fashion using data from one site and then calculated for the remaining data using the fixed formula. ROC curve analyses were performed on data from individual sites and all sites combined.

### Serum fractionation:

25 ul of supernatant were mixed with 37.5 ul of a denaturing buffer (U9: 9 M urea, 2% CHAPS, 50 mM Tris pH 9.0) and vortexed for 30 minutes at 4 degrees. Add 37.5 ul of 50 mM Tris (pH9) to give a total volume of 100 ul. For each sample, 100 ul of Q Ceramic HyperD 20 anion exchange resin as 50% suspension was equilibrated in 100 ul of 50 mM Tris (pH9) first, then U1 buffer (U9 that was diluted 1:9 in 50 mM Tris pH 9.0) three times. 100 ul of the denatured serum was applied to the resin and allowed to bind for thirty minutes at 4 degrees. The unbound material was collected and then 75 ul of wash buffer 1 (50 mM Tris-HCl + 0.1% OGP + 50 mM Sodium Chloride, pH 9) was added to the resin. The resin was agitated for 10 minutes on a Micromix. This wash was collected and combined with the unbound material (flow through; fraction 1). Fractions were then collected in a stepwise pH gradient using two times 75 ul each aliquots of wash buffers at pH 7, 5, 4, 3, and organic solvent. Each time the resin was agitated for 10 minutes on a Micromix. This led to the collection of a total of six fractions. The buffers are as follows: Wash Buffer 2: 50 mM HEPES + 0.1% OGP 50 mM Sodium Chloride (pH 7); Wash Buffer 3: 100 mM Sodium Acetate + 0.1% OGP + 50 mM Sodium Chloride (pH 5); Wash Buffer 4: 100 mM Sodium Acetate + 0.1% OGP + 50 mM Sodium Chloride (pH 4); Wash Buffer 5: 50 mM Sodium Citrate + 0.1% OGP + 50 mM Sodium Chloride (pH 3); Wash Buffer 6: 33.3% 2-propanol/ 16.7% acetonitrile/ 0.1% trifluoroacetic acid. Fractionation was performed on a Tecan Aquurius 96 (Tecan) and a Micromix shaker (DPC). A sample of control pooled human serum (Intergen) was processed identically in one well of each column of samples.

### Chip binding:

20 ul of each fraction was first pH adjusted with 20 ul of different buffers and then bound to IMAC and CM10 ProteinChip arrays. For IMAC arrays, the spots were charged with copper and rinsed and equilibrated. Fractions 1 and 2 were mixed with 20 ul of IMAC binding buffer (100 mM sodium phosphate pH 7.0 containing 500 mM NaCl); fractions 3-6 were mixed with 100 mM Tris HCl pH 10. For CM10, fractions 1, 2 and 3 were mixed with 20 ul of 100 mM acetic acid; fractions 4, 5 and 6 were mixed with 20 ul of CM10 binding buffer (100 mM Na Acetate, pH 4.0). Binding was allowed to occur for 120 minutes at room temperature. Chips were then washed two times with 150 ul binding buffer and then twice with 200 ul water. The matrix used was SPA (add 400 ul of 50% acetonitrile and 0.5% TFA to one tube, mix 5 minutes). Each spot was deposited with 1 ul of matrix twice. Chip binding was performed on a Tecan Aquurius 96 (Tecan) and a Micromix shaker (DPC).

### Data acquisition and analysis:

ProteinChip arrays were read on PCS4000 instruments using CiphergenExpress software version 3.0. Instruments were monitored weekly for performance using insulin and immunoglobulin standards. Each chip was read at two laser energies, low and high. Spectra were organized and baseline subtracted. Spectra were externally calibrated using a set of calibrants. Spectra were then normalized to total ion current according to the following parameters: for chips containing SPA, the low energy starting mass was 2000 M/Z; the high energy starting mass was 10000 M/Z. For peak clustering, the signal to noise ratio was set at 3.

### Example 2:

### Chromatographic assay of ovarian cancer markers

Add 50ul 50% suspension of IDA-Ni (II) beads (Biosepra IMAC Hypercel, charged with 0.1M NiSO4) to 96-well filter plate. Transfer IDA-Ni slurry to a plastic beaker and keep slurry well mixed by hand or on magnetic stir plate at low speed during dispensing. Use pipet tips with large orifice to dispense beads.

Wash beads three times, each with 200ul 0.02% (w/v) Triton X100 PBS (2x). 5ul serum + 7.5ul 9M urea 2% (w/v) CHAPS 50mM Tris HCl pH9 in v-bottom 96-well plate, RT vortex 2 min.

Dilute with 150ul 0.02% (w/v) Triton X100 PBS (2x) with protease inhibitor cocktail (Roche, no EDTA, 1 tablet per 50 ml).

Add to IDA-Ni plate, RT shake for 30 min on Micromix shaker (settings: 15,6,30).

Vacuum filter on vacuum manifold.

Wash plate with 200ul of 0.02% TX100 PBS (2x) eight times, no mixing.

Gently blot dry bottom of filter plate on Kimwipe (remember to dry edges of filter membrane).

Elute with 75ul 10mM imidazole 1M urea 0.1% (w/v) CHAPS 0.3M KCl 100mM TrisHCl pH7.5 four times, mix 10 min each time (15,6,10). Vacuum filter to collect in 0.45ml v-bottom 96-well plate.

### IMAC30 chip binding in bioprocessor:

Add 50ul 50mM CuSO4 to each well. Shake for 10min on Micromix (15,5,10).

Empty wells. Add 150ul water and empty wells.

Add 50ul 50mM NaOAc pH4 to each well. Shake for 5min.

Empty wells. Add 150ul water and empty wells.

Equilibrate 2 times 200ul 1M urea 0.1% CHAPS 0.3M KCl 100mM Tris HCl pH7.5.

Shake for 5 min each (15,5,5) on Micromix.

Mix eluate plate at low speed for 1 minute on Micromix.

Add 40ul of eluate to 150ul (final imidazole 2.1mM) of 1M urea 0.1%CHAPS 0.3M KCl 100mM Tris HCl pH7.5 on IMAC30-Cu (II) chip. Seal with tape and vortex 60 min (15,5,60) at RT.

Wash 1 time with 200ul 1M urea 0.1% (w/v) CHAPS 0.3M KCl 50mM TrisHCl pH 7.5, mix 5 min.

Wash 2 times with 200ul water, mix 1 min each.

Add 2 times 1ul sinapinic acid. 1 tube SPA + 200ul ACN + 200ul 1% TFA.

Read chips using low laser intensity. Focus at 14KDa.

### Q10 chip binding in bioprocessor:

Equilibrate 2 times 200ul 0.1M sodium phosphate buffer pH7.5. Shake for 5 min each (15,5,5).

Mix eluate plate at low speed for 1 minute on Micromix.

Add 40ul of eluate to 150ul (final imidazole 2.1mM) of 0.1M sodium phosphate buffer pH7.5 on Q10 chip. Seal with tape and vortex 60 min (15,5,60) at RT.

Wash 2 times with 200ul 0.1M sodium phosphate pH7.5, mix 5 min each.

Wash 1 time with 200ul water, mix 1 min.

Add 2 times 1ul sinapinic acid. 1 tube SPA + 200ul ACN + 200ul 1% TFA.

Read chips using low and high laser intensity. Focus at 14KDa.

### Preparation of IDA-Ni beads:

Measure 100ml IMAC Hypercel (Biosepra) packed gel in a graduated cylinder.

Transfer to a filter unit (0.45µm cellulose acetate). Wash beads with 500ml water.

Transfer packed beads to a 500ml round bottle.

Add 100ml 0.1M NiSO4 to beads and mix on rotator for 2 hour at RT.

Wash beads with 1000ml water in filter unit.

Wash with 500ml 2x PBS pH7.2.

Store IDA-Ni beads in 2x PBS pH7.2 as 50% slurry at 4°C.

### Materials and reagents:

IMAC Hypercel (Biosepra)
Pipet tips with large orifice 1-200ul (VWR 53503-612)
NiSO4.7H2O
PBS pH7.2, 10x (GIBCO, dilute to 2x)
Urea
CHAPS (prepare 10% (w/v) CHAPS stock solution in water)
Tris base (to prepare U9CHAPS TrisHCl pH9)
HCl for adjusting pH of Tris base
Triton X100 (prepare 1% (w/v) TX100 stock solution in water, dilute in 2x PBS to make 0.02%)
Complete protease inhibitor cocktail tablets, EDTA-free (Roche, 1 873 580)
Silent Screen filter plate 96 well, Loprodyne membrane 1.2µm pore (Nalge Nunc, 256065)
Vacuum manifold for 96-well plates
Imidazole
KCl
1M Tris HCl pH7.5 (Invitrogen)
CuSO4.5H2O
Sodium acetate and acetic acid (to make 50mM sodium acetate buffer pH4)
Sodium phosphate monobasic and dibasic (to make 0.1M sodium phosphate buffer pH7.5)
Sinapinic acid (Ciphergen Biosystems)
Acetonitrile
TFA
IMAC30 chips
Q10 chips

## Claims

1. An in vitro method for diagnosing ovarian cancer in a subject comprising:
(a) measuring β-2 microglobulin, CA125, ApoAl, transthyretin and transferrin in a biological sample from the subject; and
(b) correlating the measurement with ovarian cancer, wherein an increase in β-2 microglobulin and CA125 and a decrease in ApoAl, transthyretin and transferrin relative to a respective reference measurement for each of said biomarkers is indicative of ovarian cancer.

2. The method of claim 1, wherein the biomarkers are measured using mass spectrometry or immunoassay.

3. The method of claim 1, wherein the biomarkers are measured by capturing the biomarkers on an adsorbent surface of an SELDI probe and detecting the captured biomarkers by laser desorption-ionization mass spectrometry.

4. The method of claim 3, wherein the adsorbent is a IMAC copper adsorbent or a biospecific adsorbent.

## Patentansprüche

1. In-Vitro-Verfahren zum Diagnostizieren von Eierstockkrebs bei einem Subjekt, umfassend:
(a) Messen von β-2-Microglobulin, CA125, ApoAl, Transthyretin und Transferrin in einer biologischen Probe von dem Subjekt und
(b) Korrelieren der Messung mit Eierstockkrebs, wobei eine Zunahme von β-2-Microglobulin und CA125 und eine Abnahme von ApoAl, Transthyretin und Transferrin, bezüglich einer entsprechenden Referenzmessung für jeden der Biomarker, auf Eierstockkrebs hinweist.

2. Verfahren gemäß Anspruch 1, wobei die Biomarker unter Verwendung von Massenspektrometrie oder einem Immunoassay gemessen werden.

3. Verfahren gemäß Anspruch 1, wobei die Biomarker gemessen werden, indem die Biomarker an einer Oberfläche eines Adsorptionsmittels einer SELDI-Sonde eingefangen werden und die eingefangenen Biomarker durch Laserdesorption-Ionisationsmassenspektrometrie detektiert werden.

4. Verfahren gemäß Anspruch 3, wobei das Adsorptionsmittel ein IMAC-Kupferadsorptionsmittel oder ein biospezifisches Adsorptionsmittel ist.

## Revendications

1. Procédé in vitro pour diagnostiquer le cancer de l'ovaire chez un sujet, comprenant les étapes consistant à:
(a) mesurer le β-2 microglobuline, le CA125, l'ApoA1, la transthyrétine et la transferrine dans un échantillon biologique du sujet, et
(b) établir une corrélation de la mesure avec le cancer de l'ovaire, dans lequel une augmentation du β-2 microglobuline et du CA125 et une diminution de l'ApoA1, de la transthyrétine et de la transferrine par rapport à une mesure de référence respective pour chacun desdits biomarqueurs indiquent le cancer de l'ovaire.

2. Procédé selon la revendication 1, dans lequel les biomarqueurs sont mesurés en utilisant la spectrométrie de masse ou un immunoessai.

3. Procédé selon la revendication 1, dans lequel les biomarqueurs sont mesurés en capturant les biomarqueurs sur une surface adsorbante d'une sonde SELDI et en détectant les biomarqueurs capturés par spectrométrie de masse par désorptionionisation laser.

4. Procédé selon la revendication 3, dans lequel l'adsorbant est un adsorbant de IMAC-cuivre ou un adsorbant biospécifique.


### SEQUENCE LISTING

<110> VERMILLION, INC.
<120> BIOMARKERS FOR OVARIAN CANCER: BETA-2 MICROGLOBULIN
<130> 18311EP1
<140>
   <141> 2006-06-23
<150> US 60/693,679
   <151> 2005-06-24
<160> 6
<170> PatentIn Ver. 3.3
<210> 1
   <211> 30
   <212> PRT
   <213> Homo sapiens
<400> 1
   Met Asn Phe Arg Pro Gly Val Leu Ser Ser Arg Gln Leu Gly Leu Pro
    1               5                  10                  15
   Gly Pro Pro Asp Val Pro Asp His Ala Ala Tyr His Pro Phe
               20                  25                  30
<210> 2
   <211> 25
   <212> PRT
   <213> Homo sapiens
<400> 2
   Asp Thr His Phe Pro Ile Cys Ile Phe Cys Cys Gly Cys Cys His Arg
    1               5                  10                  15
   Ser Lys Cys Gly Met Cys Cys Lys Thr
               20                  25
<210> 3
   <211> 24
   <212> PRT
   <213> Homo sapiens
<400> 3
   Thr His Phe Pro Ile Cys Ile Phe Cys Cys Gly Cys Cys His Arg Ser
    1               5                  10                  15
   Lys Cys Gly Met Cys Cys Lys Thr
               20
<210> 4
   <211> 20
   <212> PRT
   <213> Homo sapiens
<400> 4
   Ile Cys Ile Phe Cys Cys Gly Cys Cys His Arg Ser Lys Cys Gly Met
    1               5                  10                  15
   Cys Cys Lys Thr
               20
<210> 5
   <211> 119
   <212> PRT
   <213> Homo sapiens
<400> 5
   Met Ser Arg Ser Val Ala Leu Ala Val Leu Ala Leu Leu Ser Leu Ser
    1               5                  10                  15
   Gly Leu Glu Ala Ile Gln Arg Thr Pro Lys Ile Gln Val Tyr Ser Arg
               20                  25                  30
   His Pro Ala Glu Asn Gly Lys Ser Asn Phe Leu Asn Cys Tyr Val Ser
           35                  40                  45
   Gly Phe His Pro Ser Asp Ile Glu Val Asp Leu Leu Lys Asn Gly Glu
       50                  55                  60
   Arg Ile Glu Lys Val Glu His Ser Asp Leu Ser Phe Ser Lys Asp Trp
   65                  70                  75                  80
   Ser Phe Tyr Leu Leu Tyr Tyr Thr Glu Phe Thr Pro Thr Glu Lys Asp
                   85                  90                  95
   Glu Tyr Ala Cys Arg Val Asn His Val Thr Leu Ser Gln Pro Lys Ile
               100                 105                 110
   Val Lys Trp Asp Arg Asp Met
           115
<210> 6
   <211> 22
   <212> PRT
   <213> Homo sapiens
<400> 6
   Phe Pro Ile Cys Ile Phe Cys Cys Gly Cys Cys His Arg Ser Lys Cys
    1               5                  10                  15
   Gly Met Cys Cys Lys Thr
               20